# EUROPÄISCHE PATENTSCHRIFT

(11) **EP 1 688 106 B1**
(45) Veröffentlichungstag und Bekanntmachung des Hinweises auf die Patenterteilung: **14.05.2014**
(21) Anmeldenummer: 06008529.7
(22) Anmeldetag: 25.07.2003
(51) Int. Cl.: A61F 5/01

(54) **Orthopädische Vorrichtung zur Korrektur von Zehenfehlstellungen**
Orthopedic device for correcting abnormal positions of toes
Dispositif orthopédique pour corriger des malpositions des orteils

(30) Priorität: 30.08.2002 DE 10240121
(43) Veröffentlichungstag der Anmeldung: 09.08.2006
(62) Teilanmeldung aus: 03790816.7
(73) Patentinhaber: Huber, Vitus Maria, 80637 München (DE); Hallufix AG, 80339 München (DE)
(72) Erfinder: Huber, Vitus Maria, 80637 München (DE); Krauss, Axel, 81541 München (DE)
(74) Vertreter: Leske, Thomas

(56) Entgegenhaltungen:
- CH-A- 128 592
- DE-A1- 4 014 728
- DE-B- 1 285 672
- DE-C- 322 651
- DE-C- 369 381
- DE-C- 587 233
- DE-U- 1 881 215
- DE-U- 8 902 545
- US-A- 892 412
- US-A- 1 183 062
- US-A- 1 213 786
- US-A- 1 746 865
- US-A- 5 542 774

## Beschreibung

Die Erfindung betrifft eine orthopädische Vorrichtung zur Korrektur von Zehenfehlstellungen nach dem Oberbegriff des Anspruchs 1.

Aus der DE 100 34 354 A1 ist eine Fußbandage zur Behandlung von Fehlstellungen des Großzehs, beispielsweise von Hallux valgus, bekannt. Sie weist eine in Fußlängsrichtung dehnbare Aufnahmeeinrichtung für den Großzeh auf, welche mit einem freien Ende an einem im Bereich des Mittelfußes angeordnete, diesen umgebende Ringbandage angebunden ist, sodass auf den Großzeh eine redressierende Kraft in Richtung der anatomischen Sollstellung des Zehs wirkt.

Solche Vorrichtungen werden erfahrungsgemäß nur ungern und unzuverlässig vom Patienten angelegt, weil sie stark auftragen und im normalen Schuhwerk als hinderlich oder nach längerer Tragedauer sogar lästig oder schmerzhaft empfunden werden. Hierdurch ist der Behandlungserfolg bei Anwendung einer derartigen Bandage nicht sichergestellt.

Aus US 9 33 423 ist eine Schutzvorrichtung für den Ballen des Großzehgelenks bekannt, welches selbiges beim Tragen im Schuh vor Druck durch den Schuh schützen soll. Diese Vorrichtung besteht aus einer mehrfach gekrümmten schalenförmigen Teilbereiche aufweisenden Fußschiene aus Metall, welche den Ballen topfförmig umgibt und sich teilweise oder vollständig bis unter die Fußsohle erstreckt. Diese Vorrichtung ist einteilig, erstreckt sich vom Großzeh bis in den Mittelfußbereich und soll unter dem Ballen an der Fußsohle in vertikaler Richtung so flexibel sein, dass sie sich beim Gehen beim Tragen im Schuh durch einen Einschnitt soweit biegen kann, dass der Fuß im Wesentlichen abrollen kann. In horizontaler Richtung ist diese Vorrichtung dagegen steif. Die vom selben Erfinder stammende US 1 183 062 verbessert vorstehende Vorrichtung dahingehend, dass die schalenförmige Form der Vorrichtung im Bereich unter dem Fuß anstelle des bekannten Sollbiegebereiches ein Scharniergelenk einsetzt, so dass die Vorrichtung in zwei Teile, ein Ballenteil und ein Zehenteil, geteilt ist, welche durch dieses Scharniergelenk verbunden sind. Das Ballenteil ist tassen- oder behälterförmig für das Großzehengelenk ausgebildet. Die Vorrichtung besteht aus einem steifen Material, welche steif gegen horizontales Verbiegen derart ausgebildet ist, dass der Ballen im Schuh und der Zeh gegen eine schmerzvolle seitliche Bewegung geschützt sind. Das steife Material nimmt den Druck des Schuhs auf und schützt das Großzehengelenk und den Zeh perfekt gegen den Druck des Schuhs. Für diese bekannte Ballenschutzvorrichtung ist es nötig, dass in einem relativ großen Bereich die Metallplatte unter den Fuß geführt ist, damit das Scharniergelenk entsprechend angebracht und funktionstüchtig benutzt werden kann. Das Scharniergelenk ist dabei so angeordnet, dass es parallel zur Flexions-Extensions-Achse des Großzehgelenks verläuft. Diese Vorrichtung dient zum Schutz des Ballens des Großzehgelenks beim Tragen. Diese bekannte Schutzschiene wird im Großzehbereich und im Fersenbeinbereich mittels einstellbarer Bänder, welche zur Behandlung nach und nach angezogen werden, an den Fuß geschnallt, sodass durch das allmähliche Nachziehen der Bänder das Großzehengelenk geradegerichtet wird. Nachteilig bei diesen bekannten Vorrichtungen ist, dass die Gelenkbewegung in der Vorrichtung unter dem Fuß bereitgestellt wird. Die Vorrichtung ist steif gegen horizontales Verbiegen und das Geraderichten der Zehenfehlstellung erfolgt allmählich durch schrittweises Anziehen der Bänder.

In DE 322 651 ist eine Vorrichtung zum Schutz eines Fußballens und Geraderichten der Zehen in Form einer Schuheinlage oder Einlegesohle mit einer elastischen Zugschleife beschrieben. Im Bereich des Ballens des Großzehgelenks weist die Schuheinlage ein gewölbtes Schalengelenk auf, wobei die ineinander greifenden Schalen mittels laschenartiger Fortsätze mit einem seitlich hochstehenden Rand der Schuheinlage in genieteter Form verbunden sind. Die Schuheinlage wird hierdurch derart abgestützt, dass die Schuheinlage der an der im Vorderteil der Schuheinlage angeordneten elastischen Zugschleife hinreichenden Rückhalt bietet. Die Schuheinlage bildet ein Widerlager für die elastische Zugschleife.

Aus US 1 213 786 ist eine Schiene aus einem Federmaterial bekannt, welche auf den Fußballen fixiert ist und über ein Gelenk mit einer steifen Unterfuß-Platte verbunden ist. Dieses Gelenk, welches eine Verbindung zwischen einer Bandage mit angebrachter Unterfuß-Platte und einer sich von der Großzehe über den Ballen bis kurz hinter den Ballen und zu besagtem Gelenk erstreckenden Schiene darstellt, dient zur Ausrichtung der Bandage am Fuß und noch im Auslaufbereich des Ballens. Im Großzehenbereich ist eine weitere Bandage zur Festlegung des Großzehs an der Schiene vorgesehen. Auch bei dieser Schiene ist ein Tragen im normalen Schuhwerk bei längerer Tragedauer und möglichst beschwerdefreiem Abrollen nicht gegeben.

Aus der DE 1 881 215 U1 ist eine Ballenschiene bekannt, welche als Biegefeder wirkend entlang der Fußinnenseite verläuft und an ihrem zehenseitigen Ende eine Ringöse aufweist, welche zur Aufnahme des Großzehs dient. Am gegenüberliegenden Ende weist die Ballenschiene eine Umbiegung auf, welche sich um die Ferse legen lässt. Hierdurch lässt sich eine Großzehe aus einer eingebogenen Zehenfehlstellung in die Normalstellung verbringen. Diese Schiene weist erhebliche Nachteile auf, z. B. wird sie von den Trägern als extrem unbequem im Tragekomfort empfunden, sodass diese nur sehr ungern angelegt wird, was den Behandlungserfolg nicht gewährleistet.

Aus dem deutschen Gebrauchsmuster DE 8 902 545.8 U1 ist eine Vorrichtung zur Behandlung von Großzehen bekannt, welche einen Strumpf mit einem den Großzehen umgebenden Großzehenfach aufweist und eine entlang der Fußinnenseite verlaufende Schiene aufweist, welche in einer auf den Socken aufgenähten Tasche angeordnet ist. Eine derartige Vorrichtung zur Behandlung von Großzehen ist zur Behandlung in der Nacht- bzw. Schlafzeit gedacht. Nachteilig ist, dass die Bewegungsfreiheit der geschienten Großzehe in der Flexion-Extension-Bewegungsrichtung des Großzehs unterbunden ist. Diese Vorrichtung eignet sich somit nicht für eine Dauerbehandlung. Ein Tragen dieser Vorrichtung in einem Schuh ist für einen Patienten sehr unangenehm und schränkt die Bewegungsfreiheit stark ein.

In DE 369 381 ist eine Vorrichtung zur Korrektur von Zehen-Fehlstellungen beschrieben. Diese Vorrichtung weist zwei Befestigungseinrichtungen in Form einer Lederschlaufe an der Großzehe und eines Lederriemens hinter dem Ballen des Großzehengelenks sowie eine Schiene auf, welche sich längs eines Teils der Fußinnenseite erstreckt. Diese Schiene weist ein Gelenk auf, dessen Achse senkrecht zur Sohlenfläche des Fußes verläuft. Im Bereich des Ballens ist an dem Teil der Vorrichtung, welcher sich von dem Gelenk in Richtung der Großzehe erstreckt, eine Stellschraube vorgesehen. Mit dieser Stellschraube wird der vom Gelenk in Richtung Großzehe vorhandene Fortsatz der Schiene um das Gelenk verschwenkt und dadurch eine auf die Großzehe ausgeübte Zugkraft eingestellt. Ein weiterer Verstellmechanismus dient dazu, die Lederschlaufe für den Großzeh je nach Größe des Fußes und anatomischer Gegebenheit in Längsrichtung des Fußes zu verstellen, wobei der sich vom Gelenk in Richtung Großzehe erstreckende Verstellmechanismus selbst biegesteif ausgeführt ist. Eine elastische Schiene erstreckt sich von dem Gelenk bis in den Fersenbereich und umgreift diesen. Ein permanentes Tragen im Schuh ermöglicht diese bekannte Schiene nicht, erst recht nicht ein Abrollen beim Laufen.

Bezüglich dieser Stahlschiene ähnelt diese Vorrichtung derjenigen gemäß der aus DE 1 881 215 U1 bekannten, als Biegefeder wirkenden Ballenschiene. Diese Vorrichtung gestattet zwar, je nach Behandlungserfolg die Vorrichtung an die mit fortschreitender Behandlungsdauer sich an eine Normalstellung annähernde Großzehe zu korrigieren bzw. einzustellen, das Vorhandensein dieser Verstellmechanismen verhindert jedoch ein tägliches Tragen und erst recht ein Tragen im Schuh. Ein Abrollen des Fußes über den Ballen beim Gehen ist mit dieser Vorrichtung ebenfalls nicht möglich.

Aus US 892 412 ist des Weiteren eine Vorrichtung zur Behandlung von Fußballen zum Geraderichten der Zehe bzw. der Zehen bekannt, mittels welcher ein vergrößerter Ballen des Großzehengelenkes verkleinert werden kann. Diese Vorrichtung weist eine kurze Schiene aus einem Federmaterial auf, wobei die Vorrichtung mit Riemen oder Bändern an einer oder an zwei Zehen und dem Fußmittelteil befestigt wird. Im Bereich des Ballens weist die Vorrichtung eine schüsselartige gewölbte Form auf, um auf dem vergrößerten Ballen aufzuliegen. In seinen äußeren Abmessungen ist diese Vorrichtung zwar klein, beim permanenten Tragen ergibt sich jedoch die Schwierigkeit, dass der ohnehin geschädigte vergrößerte Ballen des Großzehengelenkes in dem ausgewölbten Teil der Schiene permanent reibt und dadurch die freizügige und dauerhafte Benutzung einer derartigen Schiene zumindest wesentlich eingeschränkt ist. Die Lederriemen weisen jeweils eine Reihe von Einstelllöchern auf, so dass sie bedarfsweise straffer gestellt oder gelockert werden können. Eine Einstellbarkeit erfolgt somit über die jeweiligen Lederriemen. Weiterhin sind Spreizvorrichtungen bekannt, die als Keil ausgebildet sind und im Zwischenraum zwischen dem Großzeh und dem zweiten Zeh angeordnet sind, sodass der Großzeh zur Fußinnenseite hin gedrückt wird. Diese Vorrichtungen haben den Nachteil, dass sie sich zur Kraftausübung an den benachbarten Zehen abstützen und somit eine Fehlstellung der Nachbarzehen verursachen oder fördern können.

Aufgabe der Erfindung ist es, eine Vorrichtung zu schaffen, mit der Valgus-Fehlstellungen von Zehen, d. h. Fehlstellungen von einer oder mehreren Zehen zur Fußaußenseite hin, behandelt werden können. Weiterhin soll die Vorrichtung angenehm tragbar, insbesondere ohne nennenswerte Beeinträchtigung im Alltag tragbar sein. Der Behandlungserfolg soll gegenüber dem Stand der Technik verbessert werden.

Diese Aufgabe wird mit einer orthopädischen Vorrichtung zur Korrektur von Zehenfehlstellungen mit den Merkmalen des Anspruchs 1 gelöst. Vorteilhafte Weiterbildungen der Erfindung sind in den Unteransprüchen angegeben.

Im Folgenden wird die Erfindung anhand der Zeichnungen beispielhaft näher erläutert. Es zeigen:
- Fig. 1: schematisch eine Unteransicht auf eine erste erfindungsgemäße Vorrichtung;
- Fig. 1a: schematisch eine Unteransicht auf eine zweite besonders bevorzugte Ausführungsform der erfindungsgemäßen Vorrichtung;
- Fig. 2: schematisch eine nicht erfindungsgemäße Ausführung der Vorrichtung in einer Ansicht auf die Fußinnenseite;

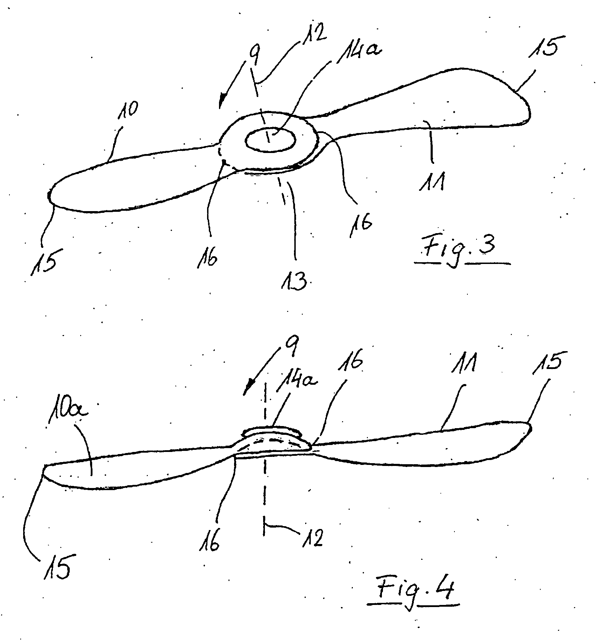

- Fig. 3: schematisch eine Gelenkschiene der erfindungsgemäßen Vorrichtung in einer perspektivischen Detailansicht;
- Fig. 4: schematisch die Gelenkschiene aus Fig. 3 in einer Draufsicht;
- Fig. 5: schematisch eine Ausschnittsansicht auf einen Vorderfußbereich (Zehe und Mittelfuß) der nicht erfindungsgemäßen Ausführungsform in einer gestreckten Stellung;
- Fig. 6: schematisch eine Ausschnittsansicht auf einen Vorderfußbereich (Zehe und Mittelfuß) der nicht erfindungsgemäßen Vorrichtung in einer abgewinkelten Stellung;
- Fig. 7: eine perspektivische Ansicht einer weiteren Ausführungsform der Gelenkschiene der erfindungsgemäßen Vorrichtung;
- Fig. 8: die Gelenkschiene gemäß Fig. 7 mit einer abgedeckten Gelenkeinrichtung;
- Fig. 9: einen Querschnitt durch die Gelenkeinrichtung der Gelenkschiene gemäß Fig. 7, entlang der Linie A-A in Fig. 7;
- Fig. 10: einen Querschnitt durch die Gelenkeinrichtung der Gelenkschiene gemäß Fig. 8 entlang der Linie A-A in Fig. 8;
- Fig. 11: einen Längsschnitt durch die Gelenkschiene gemäß Fig. 8 entlang der Linie B-B in Fig. 8;
- Fig. 12: eine perspektivische Darstellung einer weiteren Ausführungsform der Gelenkschiene der erfindungsgemäßen Vorrichtung;
- Fig. 13: eine perspektivische Darstellung der Gelenkschiene gemäß Fig. 12 mit einer abgedeckten Gelenkeinrichtung;
- Fig. 14: einen Querschnitt durch die Gelenkeinrichtung der Gelenkschiene gemäß Fig. 12 entlang der Linie A-A;
- Fig. 15: einen'Querschnitt durch die Gelenkeinrichtung der Gelenkschiene gemäß Fig. 13 entlang der Linie A-A;
- Fig. 16: einen Längsschnitt durch die Gelenkschiene gemäß Fig. 12 entlang der Linie B-B;
- Fig. 17: einen Längsschnitt durch die Gelenkschiene gemäß Fig. 13 entlang der Linie B-B;
- Fig. 18: eine weitere Ausführungsform einer Gelenkeinrichtung der Gelenkschiene in einem teilgeschnittenen Längsschnitt;
- Fig. 19: einen Querschnitt durch einen Gelenkschienenschenkel und eine zugehörige Ringbandage im Bereich des Großzehs;
- Fig. 20: einen Querschnitt durch einen Gelenkschienenschenkel und eine Ringbandage der erfindungsgemäßen Vorrichtung im Bereich des Mittelfußes;
- Fig. 21: einen zweiten Querschnitt durch einen Gelenkschienenschenkel und die zugehörige Ringbandage der erfindungsgemäßen Vorrichtung im Bereich des Mittelfußes.

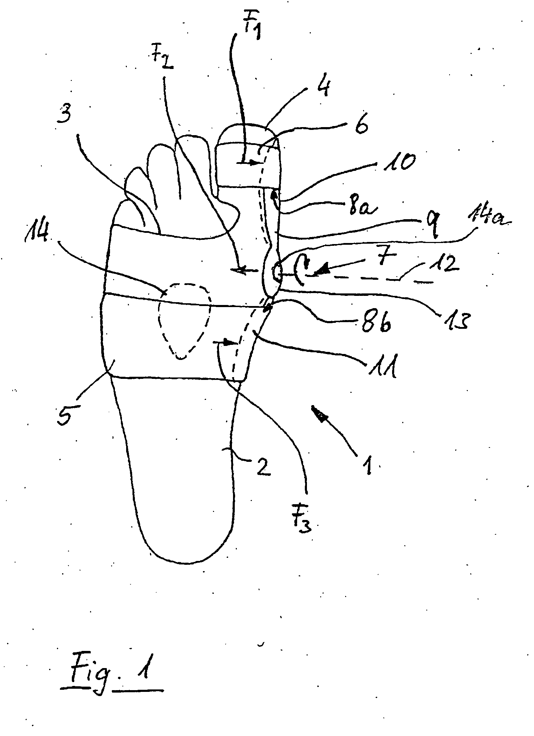

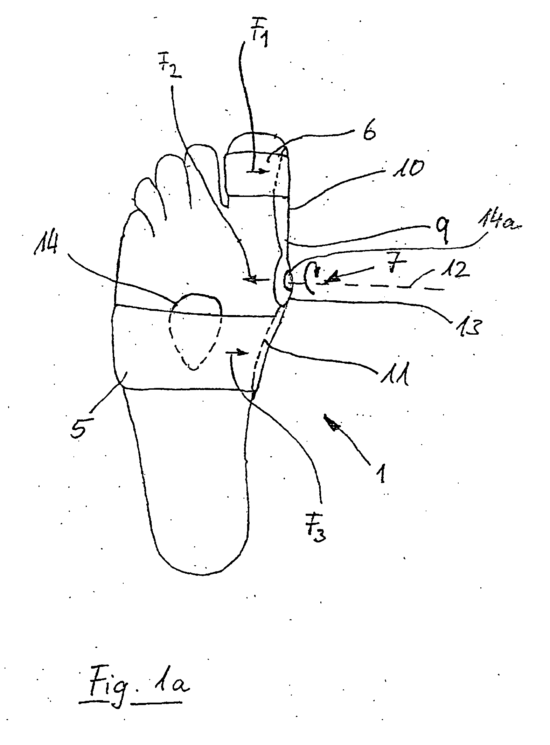

Eine erste Ausführungsform der erfindungsgemäßen Vorrichtung 1 (Fig. 1) weist einen im Zehenbereich offenen Strumpf 2 oder dergleichen Umhüllungselement für den Fuß auf. Im Bereich der Zehen weist der Strumpf 2 eine Öffnung auf, welche durch eine Begrenzungskante 3 begrenzt ist. Weiterhin weist die erfindungsgemäße Vorrichtung 1 eine Großzehenaufnahme, z.B. ein Großzehenfach 4 auf, welches mit dem Strumpf 2 einstückig verbunden oder an diesen Strumpf 2 angesetzt ist. Das Großzehenfach 4 umgibt den Großzeh umfänglich vorzugsweise vollständig und ist im Bereich des freien Zehenendes offen ausgestaltet. Bevorzugt ist das Großzehenfach 4 jedoch vorne geschlossen ausgebildet, da dies einen besseren Schutz gegen Verrutschen des Großzehenfachs 4 relativ zum Großzeh gewährleistet.

Im Bereich des Mittelfußes weist die erfindungsgemäße Vorrichtung 1 eine erste Ringbandage 5 auf, welche den Mittelfuß vorzugsweise vollständig umgibt und zweckmäßigerweise mit dem Strumpf 2 verbunden ist. Vorteilhafterweise umgibt die erste Ringbandage 5 den Strumpf 2 im Bereich des Mittelfußes außenseitig.

Das Großzehenfach 4 im Bereich des freien Endes der Großzehe umgebend ist eine zweite Ringbandage 6 angeordnet, welche den Großzeh umfänglich bevorzugt vollständig umgibt. Die Ringbandagen 5 und 6 sind vorzugsweise aus einem biegsamen, schmiegsamen, zirkulär zugstarren, d. h. in Umfangsrichtung zugstarren, Material ausgebildet, beispielsweise aus einem Gewebeband oder einem zugstarren Klebeband. Im Bereich einer Fußinnenseite 7 ist sowohl die erste Ringbandage 5 als auch die zweite Ringbandage 6 teilbereichsweise nicht mit dem Strumpf 2 bzw. dem Großzehenfach 4 verbunden, sodass zwischen den Ringbandagen 5, 6 und dem Großzehenfach 4 bzw. dem Strumpf 2 Befestigungs-/Aufnahmeeinrichtungen 8a, 8b, beispielsweise Einstecktaschen, ausgebildet sind.

Als Fußinnenseite 7 ist die Längsseite eines Fußes definiert, welche zum benachbarten Fuß weist. Die Außenseite des Fußes ist die der Innenseite gegenüberliegende Längsseite des Fußes.

Entlang der Fußinnenseite 7 erstreckt sich von der Aufnahmeeinrichtung 8b hin zur Aufnahmeeinrichtung 8a eine Biegeschiene 9. Die Biegeschiene 9 ist als Gelenkbiegeschiene ausgebildet und weist einen ersten Gelenkschienenschenkel 10 und einen zweiten Gelenkschienenschenkel 11 auf, welche um eine Schwenkachse 12 schwenkbar mittels einer Gelenkeinrichtung 13 gelenkig verbunden sind. Die Gelenkeinrichtung 13 ist bezüglich des Strumpfes 2 bzw. des Fußes derart angeordnet, dass die Schwenkachse 12 in etwa der Gelenkachse des Großzehengrundgelenks in der Flexion-Extensionsrichtung 20 , d.h. in der natürlichen Beugerichtung, also der dorsalen und plantaren Beugerichtung entspricht. Von der Gelenkeinrichtung 13 aus erstreckt sich der erste Gelenkschienenschenkel 10 bis in die Aufnahmeeinrichtung 8a. Von der Gelenkeinrichtung 13 aus erstreckt sich der zweite Gelenkschienenschenkel 11 bis in die zweite Aufnahmeeinrichtung 8b. Somit ist die Gelenkeinrichtung 13 beim Patienten in etwa im Bereich einer fußinnenseitigen, für die Hallux-valgus-Fehlstellungen typischen Ausbuchtung (Pseudoexostose) angeordnet, welche oftmals fußinnenseitig über die Sollfußumfangskontur hervorsteht. Somit wird beim Tragen einer erfindungsgemäßen Vorrichtung 1 im Bereich der Großzehe auf diese eine Kraft F1, welche in Mediolateralrichtung hin zur Fußinnenseite wirkt, ausgeübt. Im Bereich des Zehengrundgelenks wird über die Ausbuchtung eine Kraft F2 in entgegengesetzter Richtung ausgeübt. Eine aus den Kräften F1 und F2 resultierende Abstützkraft F3 wird in der Aufnahmeeinrichtung 8b von der ersten Ringbandage 5 aufgenommen.

Im Bereich der Fußsohle ist zweckmäßigerweise hinter den Grundgelenken der Zehen zur retrokapitalen Abstützung des Mittelfußes eine Pelotte 14, insbesondere eine Spreizfußpelotte eingesetzt. Diese bewirkt ein unterstützendes Aufrichten des Quergewölbes, was einen weiteren positiven Einfluss auf die Korrektur der Zehenfehlstellungen hat.

Bei der ersten Ausführungsform gemäß Fig. 1 mit den Ringbandagen 5, 6 ist auch die Verwendung eines normalen Strumpfes 2 bzw. einer normalen Strumpfstrickung möglich und zweckmäßig, da die zirkular zugstarren Ringbandagen 5, 6 eine ausreichend große Kraftübertragung gewährleisten.

In einer einfachst denkbaren besonders bevorzugten Ausführungsform (Fig. 1a) besteht die erfindungsgemäße Vorrichtung 1 lediglich aus den Ringbandagen 5, 6, der Biegeschiene 9 und ggfs. der Pelotte 14, sodass die Biegeschiene 9 zumindest teilweise direkt auf der Fußhaut des Patienten aufliegt. Diese einfachste Ausführungsform der erfindungsgemäßen Vorrichtung 1 hat sich besonders bewährt, da ein unbeabsichtigtes Verrutschen der Vorrichtung 1 relativ zum Fuß des Patienten gerade durch das Fehlen der Strumpfzwischenlage verhindert ist. Somit ist durch das Weglassen des Strumpfes überraschenderweise ein hoher Tragekomfort verwirklicht. Außerdem stellt eine Vorrichtung 1 ohne einen Strumpf die in der Herstellung kostengünstigste Ausführungsform dar. Bei der Ausführungsform gemäß Fig. 1a sind die Ringbandagen 4, 5 jeweils mit dem zugehörigen Gelenkschienenschenkel 10, 11 verbunden.

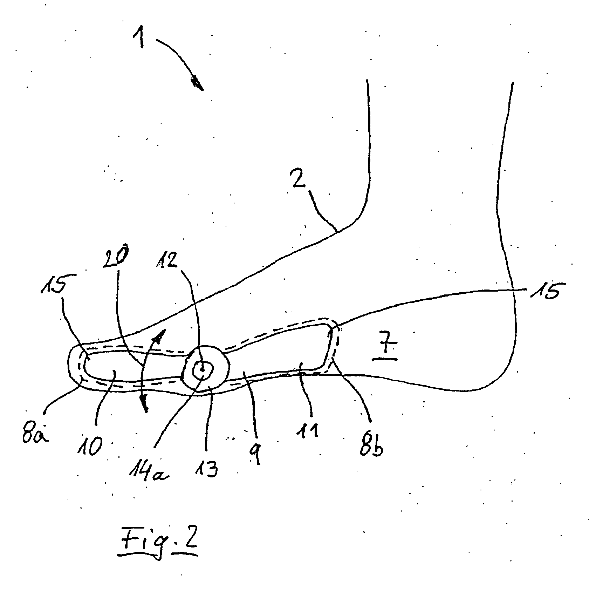

Gemäß einer nicht erfindungsgemäßen Ausführungsform der Vorrichtung (Fig. 2, 5, 6) sind die Aufnahmeeinrichtungen 8a und 8b als auf den Strumpf 2 aufgenähte oder dergleichen befestigte Taschen ausgebildet. Bei dieser Ausführungsform ist es zweckmäßig, den Strumpf 2 als so genannten Kompressionsstrumpf auszubilden, damit auch ohne die Ringbandagen 5, 6 eine ausreichend große Kraftübertragung, d. h. eine ausreichend große Einleitung der Kräfte F1 und F3 in den Fuß gewährleistet ist.

Die Gelenkeinrichtung 13 ist im Wesentlichen dreiteilig aus dem ersten Gelenkschienenschenkel 10, dem zweiten Gelenkschienenschenkel 11 und einer Gelenkschienenschenkelverbindungseinrichtung 14a, insbesondere einer Hohlniet, aufgebaut. Die Gelenkschienenschenkel 10, 11 weisen jeweils ein freies Ende 15 und ein gelenkseitiges Ende 16 auf. Die gelenkseitigen Enden 16 weisen in etwa eine Kugelkalottenraumform auf, welche zueinander derart korrespondierend ausgebildet sind, dass die jeweils gelenkseitigen Enden 16 der Gelenkschienenschenkel 10, 11 formschlüssig passend ineinanderlegbar sind. Aus den kugelkalottenförmigen gelenkseitigen Enden 16, welche mittels der Hohlniet 14a verbunden sind, ist die Gelenkeinrichtung 13 gebildet, welche durch die kugelkalottenförmige Ausgestaltung der gelenkseitigen Enden 16 eine hohe Stabilität in der Mediolateralrichtung aufweist.

Weiterhin ist durch die Ausbeulung der Gelenkeinrichtung 13 als kombinatorische Wirkung von Vorteil, dass hierdurch in einfacher Art und Weise eine individuelle Anpassung der Gelenkeinrichtung 14a an die Fußkontur des Patienten im Bereich des Großzehengrundgelenks möglich ist. Dies ist besonders vorteilhaft, da im Bereich des Großzehengrundgelenks bei Vorliegen einer Hallux-valgus-Fehlstellung oftmals ballenförmige Ausbuchtungen (d. h. eine sogenannte Pseudoexostose) am Fuß vorliegen. In besonders bevorzugter Art und Weise ist die Kalottenform, -tiefe, -größe und der Kalottendurchmesser individuell auf den Patientenfuß abgestimmt.

Die Gelenkschienenschenkel 10, 11, welche sich von der Gelenkeinrichtung 13 weg erstrecken, sind vorteilhafterweise im Querschnitt bzw. ihrer gesamten Raumform an die Fußkontur angepasst ausgebildet. Für die Ausbildung der gelenkseitigen Enden 16 der Gelenkschienenschenkel 10, 11 sind nicht nur kugelkalottenförmige Raumformen, sondern jede Art von um die Achse 12 rotationssymmetrische, zueinander korrespondierende Raumformen, insbesondere kegelstumpfförmige oder im Querschnitt parabelförmige Raumformen, geeignet die einen Gelenkring und einen Gelenkteller darstellen.

Als Material für die Biegeschiene 9 bzw. die Gelenkschienenschenkel 10, 11 eignet sich Kunststoff, wobei sich eine Kohlefaser-verstärkte dünne Platte als besonders günstig erwiesen hat, da diese unter Hitzeeinwirkung leicht formbar ist und nach dem Abkühlen eine große Federkraft bei dünner Materialstärke aufweist. Die Steifigkeit der Biegeschiene 9 in Mediolateralrichtung ist zusätzlich durch eine unebene Querschnittsform der Schenkel 10, 11 erhöht. Somit kann mit einer erfindungsgemäßen Vorrichtung mit einer Biegeschiene 9 zum einen ein hoher Tragekomfort erreicht werden, weil die Biegeschiene 9 individuell an den Fuß des Patienten anpassbar sind, und zum anderen wird hierdurch eine erhöhte Federhärte in Mediolateralrichtung erreicht, sodass im Wesentlichen hieraus resultierend ein überraschenderweise hoher Behandlungserfolg realisiert werden kann.

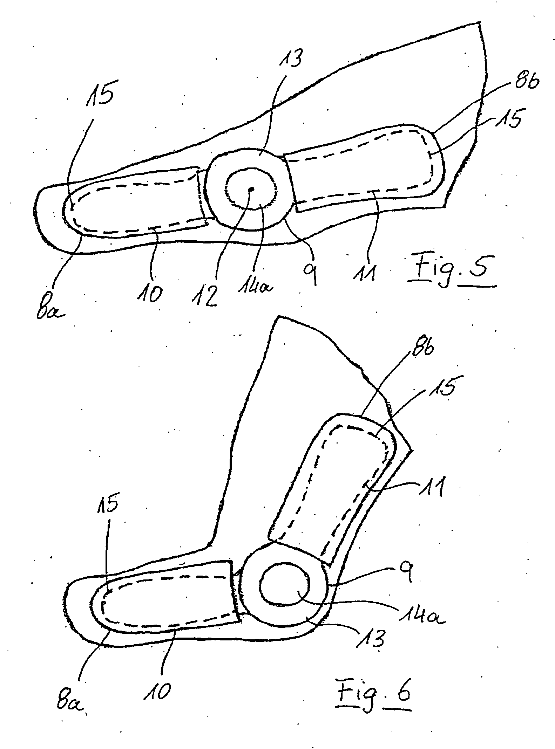

Weiterhin ist für eine erfindungsgemäße Vorrichtung 1 gewährleistet, dass an der Fußinnenseite bzw. an der zu korrigierenden Großzehe eine nur sehr dünne Materialauflage anzubringen ist, sodass die erfindungsgemäße Vorrichtung 1 ohne nennenswerte Beeinträchtigung in üblichem Schuhwerk getragen werden kann. Die Bewegungsfreiheit der Großzehe ist dadurch ebenfalls nicht eingeschränkt, da bei der erfindungsgemäßen Vorrichtung 1 die Möglichkeit besteht, die Zehe in der natürlichen Flexion-Extensionsrichtung 20 zu bewegen bzw. in diese Richtung ihre Bewegungsfreiheit nicht eingeschränkt ist (vgl. Fig. 5, 6). Somit eignet sich diese Vorrichtung 1 insbesondere für eine Dauerbehandlung sowohl tags als auch nachts, da der Patient keine nennenswerte Behinderung durch die Vorrichtung 1 verspürt.

Hinsichtlich ihrer Querschnittsraumform sind die Gelenkschienenschenkel 10, 11 uneben, d. h. beispielsweise gebogen mit konstanter Dicke aus einem Plattenmaterial geformt, wobei die Formgebung an die individuelle Fußkontur des Patienten angepasst ist. Weiterhin ist es selbstverständlich möglich, die Gelenkschienenschenkel 10, 11 in ihrem Querschnitt linsenförmig, insbesondere derart linsenförmig auszubilden, dass die Materialstärke zu den Rändern der Gelenkschienenschenkel 10, 11 hin abnimmt, sodass die geometrische Anpassung der Biegeschiene 9 an den Patientenfuß individuell weiter verbessert ist.

Gemäß einer weiteren Ausführungsform (nicht gezeigt) ist die erfindungsgemäße orthopädische Vorrichtung 1 zur Korrektur von Fehlstellungen mehrerer benachbarter Zehen weitergebildet, indem z. B. von der Ringbandage 6 um den Großzeh Zugmittel in Mediolateralrichtung abgehen und auf ein oder mehrere benachbarte Zehen einwirken, welche beispielsweise ebenfalls von einer Zehenringbandage umgeben sind. Somit kann die Korrekturkraft der Biegeschiene 9 in Mediolateralrichtung in einfacher Art und Weise vom Großzeh auf benachbarte Zehen übertragen werden. Durch geeignete Wahl der Materialstärke für die Gelenkschienenschenkel 10, 11 sowie durch eine geeignete Wahl eines Umfangsbeschnitts dieser Gelenkschienenschenkel 10, 11 sowie durch eine einfache Anpassbarkeit der Federvorspannung der Biegeschiene 9 in Mediolateralrichtung kann in einfacher Art und Weise Einfluss auf die Federhärte und somit auf die Zehenstellungs-korrigierende Kraft F1 genommen werden.

Eine individuelle Anpassung der korrigierenden Kraft F1 an spezifische Bedürfnisse des Patienten ist somit mit einfachen, in jeder orthopädischen Werkstatt vorhandenen Mitteln, z. B. durch Anpassung der Umfangskontur der Schenkel in 10, 11 oder der Veränderung der Querschnittskontur der Schenkel 10, 11 und/oder der Gelenkeinrichtung 13 möglich.

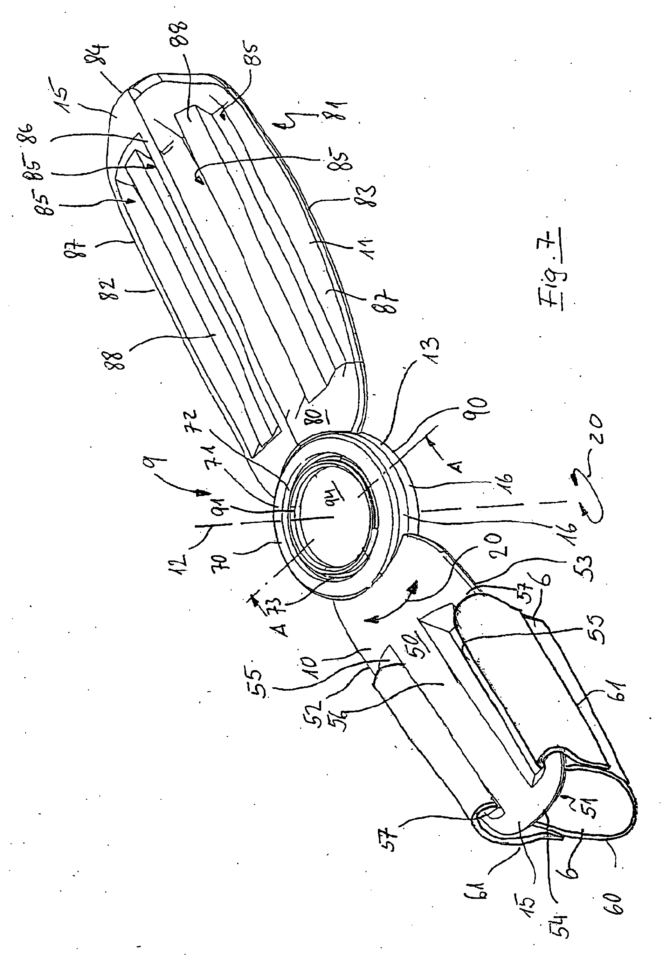

Eine weitere Ausführungsform der Gelenkschiene 9 der erfindungsgemäßen Vorrichtung 1 (Fig. 7) weist ebenfalls einen ersten Gelenkschienenschenkel 10 und einen zweiten Gelenkschienenschenkel 11 auf, welche mittels einer Gelenkeinrichtung 13 um eine Schwenkachse 12 schwenkbar gelenkig miteinander verbunden sind. Der erste Gelenkschienenschenkel 10 ist im Falle der Anordnung der Vorrichtung 1 im Bereich des Großzehs des Patienten an der Fußinnenseite angeordnet. Der zweite Gelenkschienenschenkel 11 ist in diesem Fall im Bereich des Mittelfußes des Patienten an der Fußinnenseite angeordnet. Der erste Gelenkschienenschenkel 10 weist eine vom Fuß wegweisende Außenseite 50 und eine zum Fuß hinweisende Innenseite 51 auf. Der erste Gelenkschienenschenkel 10 weist eine in Längs- und Querrichtung gewölbte, im Wesentlichen plattenförmige Raumform mit einer ersten Längsbegrenzungskante 52 und einer zweiten Längsbegrenzungskante 53 sowie einer Schmalbegrenzungskante 54 auf. Benachbart zu den Längsbegrenzungskanten 52, 53 verlaufend besitzt der Gelenkschienenschenkel 10 schlitzförmige Öffnungen 55, sodass ein Mittelsteg 56 und Randstege 57 gebildet sind.

Die Wölbungen des Gelenkschienenschenkels 10 in seiner Längs- und Querrichtung sind an die anatomischen Gegebenheiten eines Fußes angepasst.

Zur Befestigung des ersten Gelenkschienenschenkels 10 an der Großzehe des Patienten ist die Ringbandage 6 vorgesehen, welche mit dem Gelenkschienenschenkel 10 verbunden ist. Die Ringbandage 6 besitzt einen Schlaufenkörper 60 und freie Enden 61. Der Schlaufenkörper 60 dient zur Umschlingung der Großzehe des Patienten. Die freien Enden 61 sind von der Innenseite 51 her durch die schlitzförmigen Öffnungen 55, diese durchgreifend, geführt, und die Randstege 57 umgreifend mit einer Außenseite des Schlaufenkörpers 60 lösbar befestigt. Somit ist eine sichere Befestigung des Gelenkschienenschenkels 10 an der Großzehe des Patienten und eine Anpassbarkeit der Größe des Schlaufengröße 60 an unterschiedliche Größen der Großzehen gewährleistet.

Am gelenkseitigen Ende 16 des ersten Gelenkschienenschenkels 10 weist dieser einen Gelenkring 70 auf, dessen Mittelachse die Schwenkachse 12 ist. Der Gelenkring 70 ist einstückig mit dem ersten Gelenkschienenschenkel 10 verbunden. Der Gelenkring 70 besitzt eine Außenseite 71, eine Innenseite 72 sowie eine sich von der Innenseite 72 ein Stück in Richtung zur Schwenkachse 9 erstreckende Ringstufe 73 auf.

Der zweite Gelenkschienenschenkel 11 besitzt eine Außenseite 80 und einen Innenseite 81 sowie eine erste Längsbegrenzungskante 82 und eine zweite Längsbegrenzungskante 83 sowie eine Schmalseitenbegrenzungskante 84. Der zweite Gelenkschienenschenkel 11 ist hinsichtlich seiner Raumform im Wesentlichen als ein in Längsrichtung und Querrichtung gewölbter plattenförmiger Körper aufgebaut, wobei die Wölbungen in Längs- und Querrichtung an die anatomischen Gegebenheiten im Bereich des Mittelfußes an einer Fußinnenseite angepasst sind. Zum freien Ende 15 hin sowie zu den Längsbegrenzungskanten 82, 83 hin ist der zweite Gelenkschienenschenkel 11 wie auch der erste Gelenkschienenschenkel 10 sich jeweils in der Materialstärke verjüngend ausgebildet, sodass z. B. eine im Querschnitt in etwa linsenförmige Räumform ausgebildet ist. In etwa parallel zu den Längsbegrenzungskanten 82, 83 weist der zweite Gelenkschienenschenkel 11 schlitzförmige Öffnungen 85 auf, sodass ein Mittelsteg 86 sowie Randstege 87 und zwischen den Randstegen, 87 und dem Mittelsteg 86 angeordnete Zwischenstege 88 ausgebildet sind.

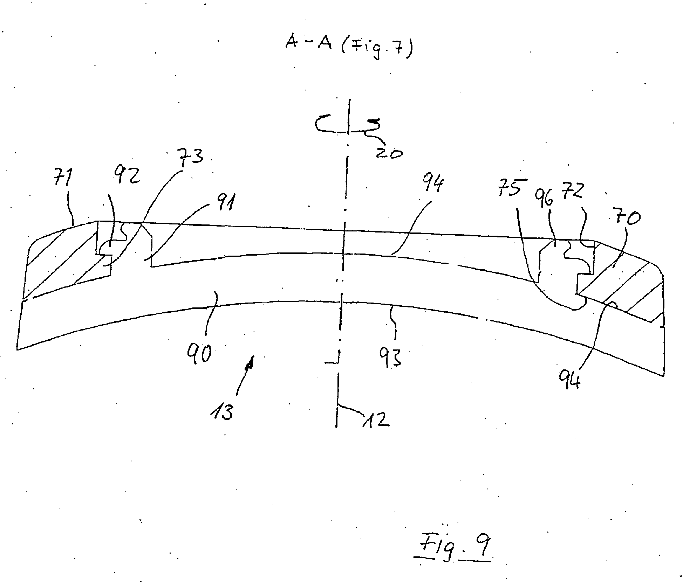

Das gelenkseitige Ende 16 des zweiten Gelenkschienenschenkels 11 ist mit dem Gelenkschienenschenkel 11 einstückig verbunden und ist als Gelenkteller 90 ausgebildet, welcher mit dem Gelenkring 70 des ersten Gelenkschienenschenkels 10 die Gelenkeinrichtung 13 bildet. Wenn die erfindungsgemäße Vorrichtung von einem Patienten getragen wird und an dessen Fuß befestigt ist, ist der Gelenkteller 90 der Fußhaut zugewandt und liegt auf dieser auf. Der Gelenkring 70 ist außenseitig gegen den Gelenkteller 90 gesetzt. Der Gelenkteller 90 weist konzentrisch zur Ringöffnung des Gelenkrings 7 einen Ringsteg 91 auf, welcher sich vom Gelenkteller 90 ein Stück in Richtung des Innenraums des Gelenkrings 70 erstreckt. Der Ringsteg 91 sitzt spielfrei oder nahezu spielfrei innerhalb der Ringstufe 73 des Gelenkrings 70, sodass eine radiale Führung des Gelenktellers 90 und des Gelenkrings 70 zueinander gewährleistet ist. Zur axialen Festlegung des Gelenkrings 70 bezüglich des Gelenktellers 90 sind an den Ringsteg 91 Rästnasensegmente 92 (Fig.9) angeformt, welche mit einer Oberseite der Ringstufe 73 zusammenwirken und somit eine axiale Festlegung der Gelenkschienenschenkel 10 und 11 zueinander gewährleisten. Die Anordnung der Rastnasensegmente 92 wird im Zusammenhang mit der Fig. 9 näher beschrieben werden. Um ein sicheres Fügen der Gelenkeinrichtung durch Aufstecken des Gelenkrings 70 auf den Gelenkteller 90 und Verrasten der Rastnasensegmente 92 mit der Ringstufe 73 zu gewährleisten, ist es zweckmäßig, die Rastnasensegmente 92 nicht vollumfänglich am Ringsteg 91 anzuordnen, damit durch die Materialelastizität der Gelenkring 70 beim Fügen auffedern kann.

Gemäß einer besonders bevorzugten Ausführungsform (Fig. 9) weist der Gelenkteller 90 eine im Querschnitt Kalottenförmige, insbesondere Kugelkalotten-förmige Raumform auf und besitzt eine zum Fuß hingewandte Seite 93 und eine zum Gelenkring 70 hingewandte Außenseite 94, welche korrespondierend zur Innenseite 93 ebenfalls, insbesondere im Bereich außerhalb des Ringstegs 91, Kalotten-förmig oder teilbereichsweise Kalotten-förmig ausgebildet ist. Korrespondierend hierzu weist der Gelenkring 70 einer der Außenseite 71 gegenüberliegende Innenseite 75 auf, welche in ihrer Raumform derart ausgebildet ist, dass sie flächig auf der korrespondierenden Innenseite 93 des Gelenktellers 90 im Bereich außerhalb des Ringstegs 91 aufliegt.

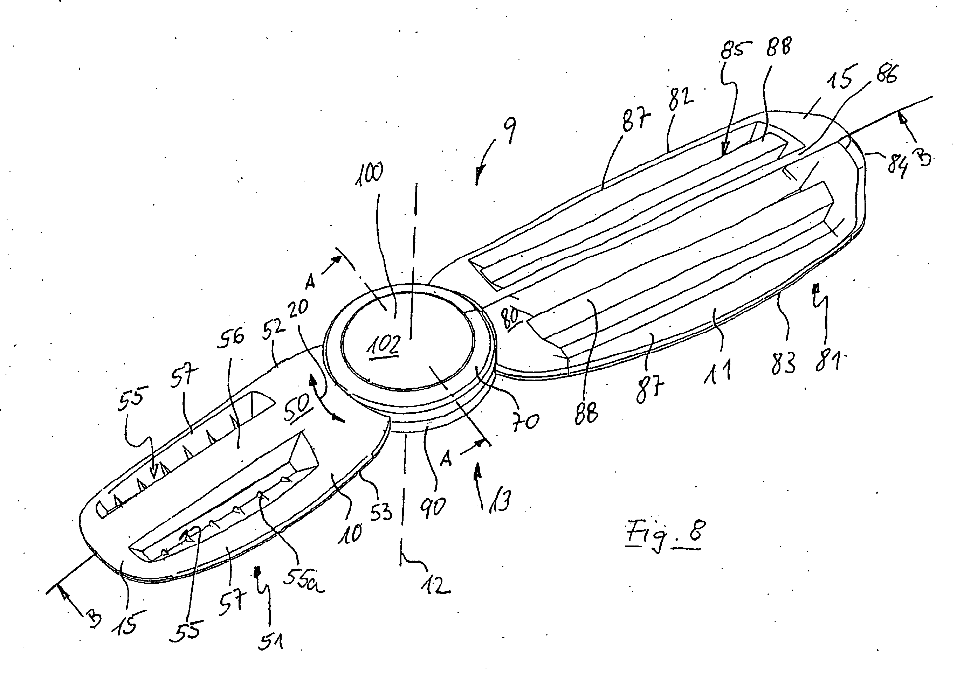

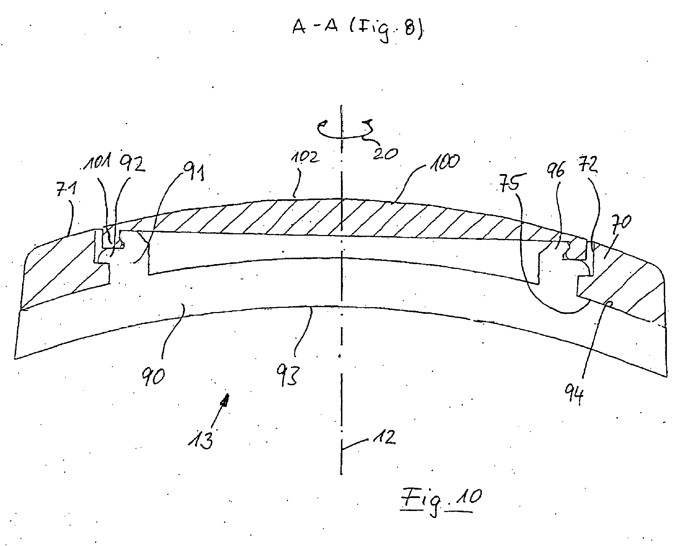

Zusätzlich zu den Rastnasensegmenten 92 weist der Ringsteg 91 an seinem freien Ende einen weiteren Rastring 96 oder Rastringsegmente auf, welche zur Befestigung einer deckelartigen Verschlusseinrichtung 100 (vergleiche Fig. 8, 10) dienen.

Die deckelartige Verschlusseinrichtung 100 weist zum Rastringsteg 96 korrespondierende Gegenrastmittel 101 auf, sodass die Verschlusseinrichtung formschlüssig mit dem Gelenkteller 90 bzw. dessen Ringsteg 91 verbindbar ist. Die Verschlusseinrichtung 100 weist eine Außenseite 102 auf, die in ihrer Raumform derart ausgebildet ist, dass ein harmonischer Übergang von der Oberseite 73 des Gelenkrings 70 gewährleistet ist. Die Verschlusseinrichtung 100 dient dazu, die offene Gelenkeinrichtung zu verschließen und somit vor Verschmutzung und/oder Beschädigung zu schützen. Bevorzugt ist die Verschlusseinrichtung 100 mit Spiel zur Innenseite 72 des Gelenkrings eingesetzt, um unerwünschte Geräusche und/oder überhöhte Reibung zu vermeiden.

Somit ist in einfacher Art und Weise eine leicht zu montierende Gelenkeinrichtung 13 geschaffen, welche eine außerordentlich hohe Biege-Belastbarkeit bei gleichzeitig geringer Materialdicke aufweist und zudem mit nur wenigen Bauteilen auskommt. Außerdem bewirkt die Ausbildung der Gelenkeinrichtung 13 nach Art eines Ringgelenks, dass in die Gelenkeinrichtung 13 hohe Biegekräfte und Biegemomente einleitbar sind, ohne deren Leichtgängigkeit zu beeinträchtigen oder derer Verschleiß übermäßig zu fördern. Dies ist vor allem auf die große Stützweite, welche durch ein außenseitiges Eingreifen der Rastnasensegmente 92 in eine innenseitige Ringstufe 72 des Gelenkrings 70 ermöglicht wird, zurückzuführen.

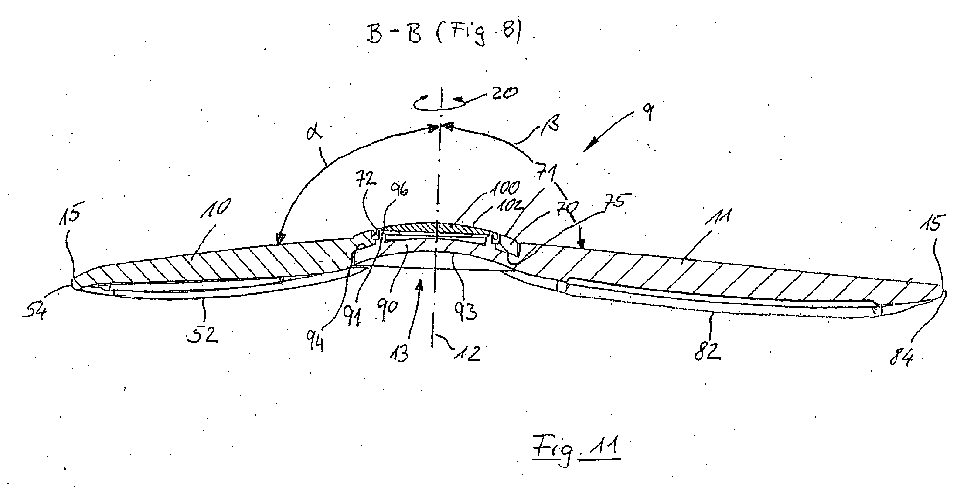

Somit ist die Gelenkschiene 9 im Wesentlichen aus lediglich zwei Einzelteilen gebildet, welche die Funktion der Schiene 9 sicherstellen. Die Verschlusseinrichtung 100 besitzt lediglich eine Verschlussfunktion und ist zur Ausbildung des Gelenks nicht notwendig. Besonders vorteilhaft bei einer solchen Ausgestaltung der Gelenkeinrichtung 13 ist, dass die im Bereich des Großzehengrundgelenks an einer eventuell vorhandenen Pseudoexostose des Patientenfußes anliegende Innenseite 93 des Gelenktellers 90 eine glatte Oberfläche aufweist, d.h. keine überstehende oder vorstehende Teilbereiche oder Kanten aufweist, was den Tragekomfort erheblich erhöht. Durch die Kalotten-förmige Ausgestaltung ist die größtmögliche Druckentlastung empfindlicher Bereiche des Fußes (Pseudoexostose) und somit eine Schmerzentlastung des Patienten sichergestellt, da die eingeleitete Druckkraft F₂ auf eine große Fläche verteilt wird. Entlang des Längsverlaufes (Fig. 11) der Gelenkschiene 9 ist dafür gesorgt, dass die Innenseite 51, die Innenseite 93 und die Innenseite 81 der Gelenkschienenbauteile 10, 11 einen stetigen, stufenfreien und geschmeidigen Verlauf aufweisen, sodass der Tragekomfort am Fuß weiter verbessert ist. Die schlitzförmigen Öffnungen 55 bzw. 85 weisen ggf. an deren Flankenflächen Verzahnungen 55a auf, welche eine Rutschsicherung für die durchzufädelnden Ringbandagen 6 darstellen.

Die Gelenkschienenschenkel 10 und 11 schließen in ihrer Längserstreckung mit der Schwenkachse 12 einen Winkel α bzw. β ein (vergleiche Fig. 11). Die Winkel α und β sind derart gewählt, dass die Gelenkschiene 9 derart an einen Patientenfuß anlegbar ist, dass die Schwenkachse 12 in etwa mit der anatomischen Gelenkachse des Großzehengrundgelenks fluchtet, sodass bei einer Schwenkbewegung des Gelenkes mit am Fuß befestigten, Gelenkschienenschenkeln 10 und 11 eine kinematisch weitgehend an die anatomischen Bedingungen des Großzehengrundgelenks angepasste Schwenkbewegung der Gelenkschiene 9 ermöglicht wird. Weiterhin sind die Winkel α und β derart gewählt, dass für eine zur Fußaußenseite hin schief gestellte Großzehe (Hallux-valgus-Stellung) eine Korrektur in Richtung hin zu einer orthopädischen Normalstellung erreichbar ist. Selbstverständlich liegt es auch im Bereich der Erfindung, die Winkel α und β derart zu wählen, dass eine seltener auftretende so genannte Hallux-varus-Stellung, d. h. eine Zehenschiefstellung, die ausgehend von der Normalstellung hin zur Fußinnenseite verschoben ist, korrigiert werden kann. Die Auswahl der Winkel α und β nach den oben genannten Kriterien ist selbstverständlich für alle in dieser Anmeldung skizzierten Ausführungsformen möglich und nicht auf die Ausführungsform gemäß den Fig. 7 bis 11 beschränkt. Je nach Grad der auftretenden Zehenschiefstellung und der erwünschten Korrekturkraft hat sich für den Winkel α ein Bereich zwischen 75° und 115° bewährt. Innerhalb dieses Bereichs ist ein Großteil der auftretenden Hallux-valgus- und Hallux-varus-Schiefstellungen korrigierbar. Selbstverständlich kann in extremen Einzelfällen auch ein Winkel α außerhalb dieses Bereichs notwendig werden. Für den Winkel β hat sich ein Winkel von etwa 70° bis 110° bewährt, um einen Großteil der üblicherweise auftretenden Hallux-valgus- und/oder Hallux-varus-Stellungen zu korrigieren. Selbstverständlich gilt auch für den Winkel β, dass er in Einzelfällen größer oder kleiner zu wählen ist.

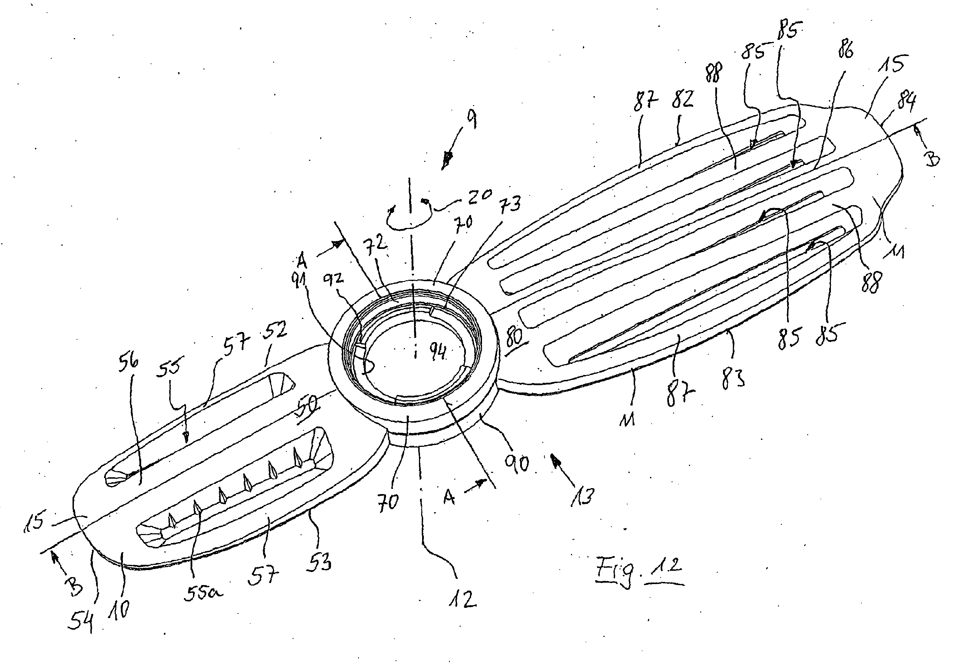

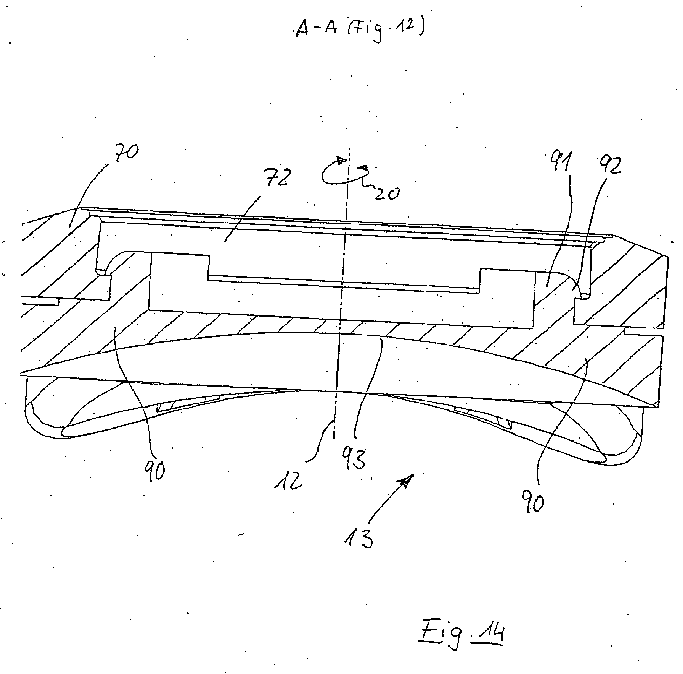

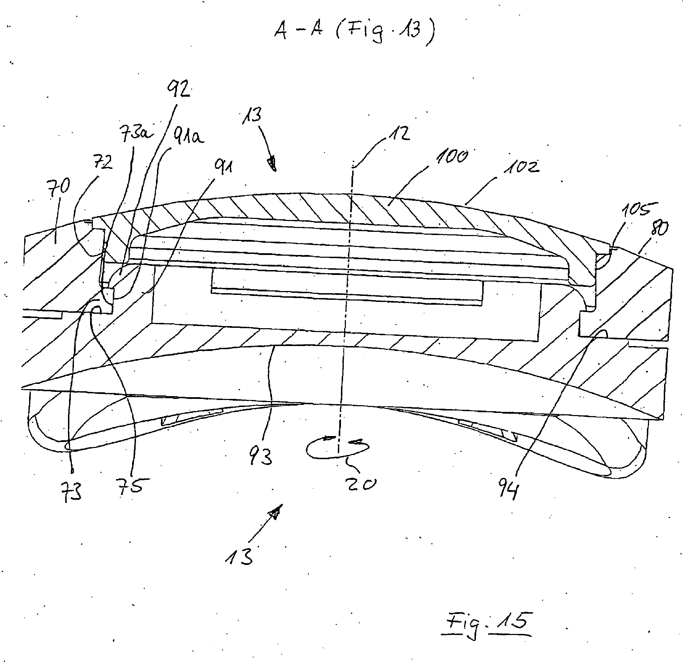

Eine weitere Ausführungsform der Gelenkschiene 9 der erfindungsgemäßen Vorrichtung 1 (Fig. 12) ist im Wesentlichen identisch aufgebaut wie die Ausführungsform gemäß den Fig. 7 und beruht hinsichtlich der Korrektur der Zehenfehlstellungen auf dem gleichen Wirkprinzip. Im Unterschied zur Ausführungsform gemäß den Fig. 7 bis 11 ist bei der Ausführungsform gemäß den Fig. 12 bis 17 lediglich die Gelenkeinrichtung 13 etwas anders gestaltet. Die Innenseite 72 des Gelenkrings 70 ist in ihrer axialen Längserstreckung etwas größer ausgebildet. Zudem verfügt der Ringsteg 91 des Gelenktellers 90 lediglich über die Rastnasensegmente 92, nicht jedoch über die aufgesetzten Rastnasensegmente 96 zur Halterung der Verschlusseinrichtung 100.

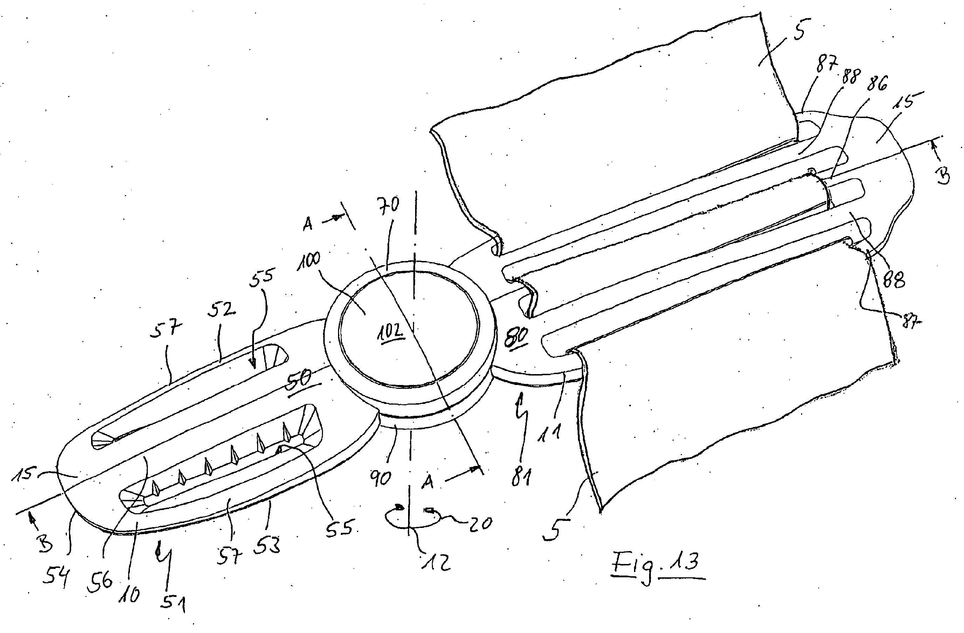

Die Verschlusseinrichtung 100 (vergleiche Fig. 13) ist derart in den Gelenkring 70 eingesetzt, dass eine Ringaußenwandung 105 der Verschlussvorrichtung 100 mit der Innenseite 72 des Gelenkrings 70 derart zusammenwirkt, dass die Verschlussvorrichtung 100 mittels eines Klemmsitzes, ggf. auch mittels eines Klebsitzes bezüglich des Gelenkrings 70 befestigt ist. Bei dieser Konstruktion ist von Vorteil, dass die Verschlusseinrichtung 100 nicht relativ zum Gelenkring 70 bewegt wird, sodass eine Scheuerwirkung auf einen ggf. die Gelenkschiene mitsamt der Gelenkeinrichtung 16 umgebenden Strumpf oder eine Innenseite des Schuhs minimiert ist. Somit ist sichergestellt, dass zwischen den Teilbereichen 50, 102, 80 der Außenseite der Gelenkschiene 9 bei Betätigung der Gelenkschiene keine Relativbewegung der Flächenabschnitte 80, 100, 70 zueinander im Bereich der Gelenkeinrichtung 13 stattfindet. Ein weiterer Unterschied ist, dass die Gelenkgleitflächen 94 des Gelenktellers 90 und die korrespondierende Fläche 75 des Gelenkrings 70 als planare Ringflächen ausgebildet sind, was eine Konstruktionsvereinfachung dahingehend darstellt, dass die radiale Führung des Gelenkrings 70 bezüglich des Gelenktellers 90 ausschließlich über eine Außenseite 91a des Ringstegs 91 und über eine Innenseite 73a der Ringstufe 73 erfolgt.

Selbstverständlich liegt es auch im Bereich der Erfindung, die Verschlusseinrichtung 100 nach Art der Ausführungsform gemäß den Fig. 12 bis 17 auf eine Ausführungsform der Gelenkeinrichtung 13 gemäß den Fig. 7 bis 12 zu übertragen. Gleiches gilt für die Ausbildung der Gleitflächen 75, 94 hinsichtlich der Übertragung des Prinzips gemäß den Fig. 7 bis 12 auf eine Gelenkeinrichtung 13 gemäß den Fig. 13 bis 17.

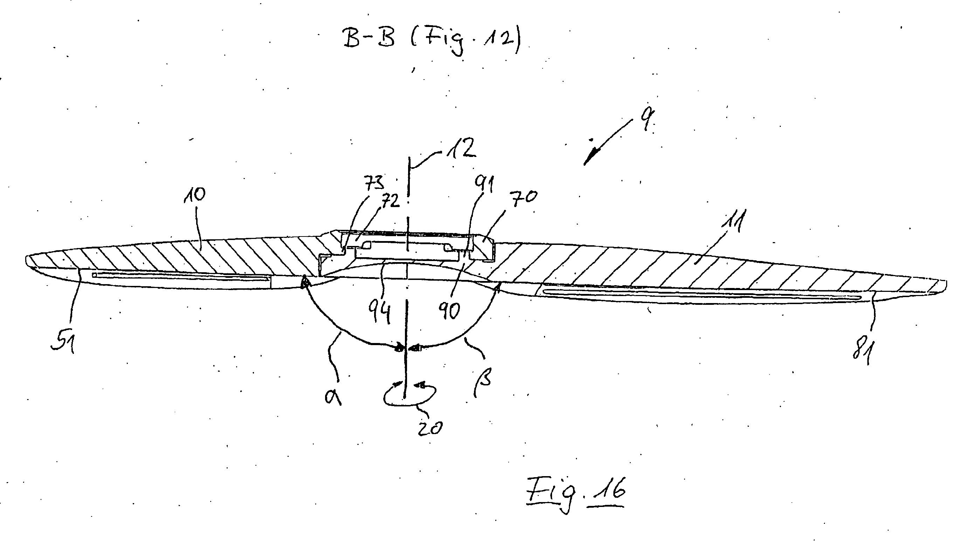

Aus einem Längsschnitt der Ausführungsform gemäß den Fig. 12 und 13. (Fig. 16) geht hervor, dass der wirksame Korrekturwinkel β zwischen der Schwenkachse 12 und den am Fuß anliegenden wirksamen Bereichen der Gelenkschiene 9 jeweils 90° ist. Weiterhin weisen die Gelenkschienenschenkel 10 und 11 in ihrem wirksamen Längsverlauf entlang ihrer Seiten 51 und 81 eine gerade Raumform auf, welche nicht in besonderem Maße an den anatomischen Verlauf eines Fußes bzw. einer Großzehe angepasst ist. Somit stellt eine derartige Ausführungsform der Gelenkschiene eine vereinfachte, aber für eine Großzahl der zu behandelnden Fälle wirkungsvolle Ausgestaltung der Gelenkschiene 9 dar. Der korrespondierende Winkel α und β in Fig. 11 wurde der Einfachheit halber zwischen der Schwenkachse 12 und der Äußenlinie eingezeichnet, was jedoch keinen wesentlichen Unterschied macht. Gemeint ist in jedem Fall der wirksame Winkel einer gedachten Gelenkschienenschenkel-Wirkachse zur Schwenkachse 12, wobei im Falle einer vollständig frei geformten, an die anatomischen Gegebenheiten des menschlichen Fußes angepassten Gelenkschiene gemäß Fig. 11 die zeichnerische Festlegung einer solchen Wirksachse nur eine grobe Schätzung sein kann. Somit wird festgestellt, dass die Winkel α und β gemäß Fig. 16 und die Winkel α und β gemäß Fig. 11 im Prinzip die gleichen Wirkwinkel darstellen sollen.

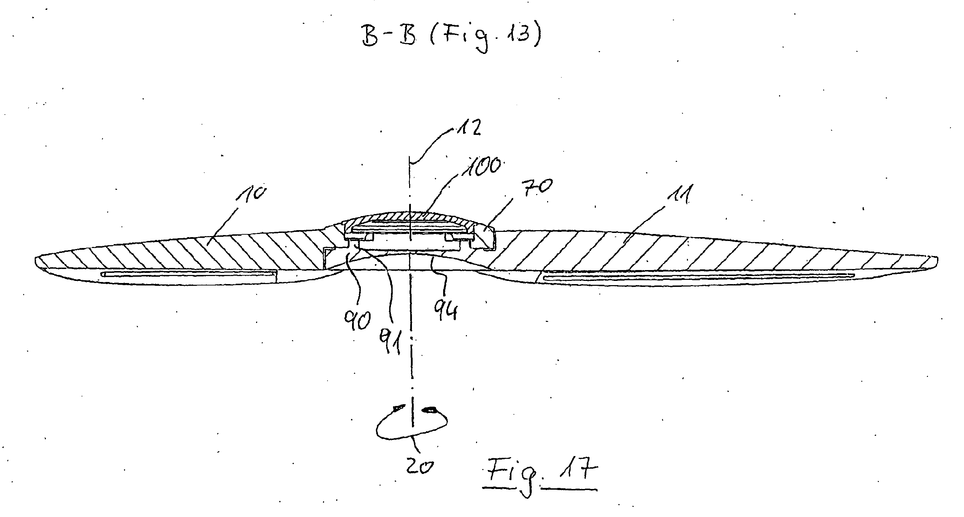

In der Längsschnittdarstellung gemäß Fig. 17 ist die Ausführungsform der Gelenkschiene 9 gemäß den Fig. 12 und 13 mit einer eingesetzten Verschlusseinrichtung 100 gezeigt.

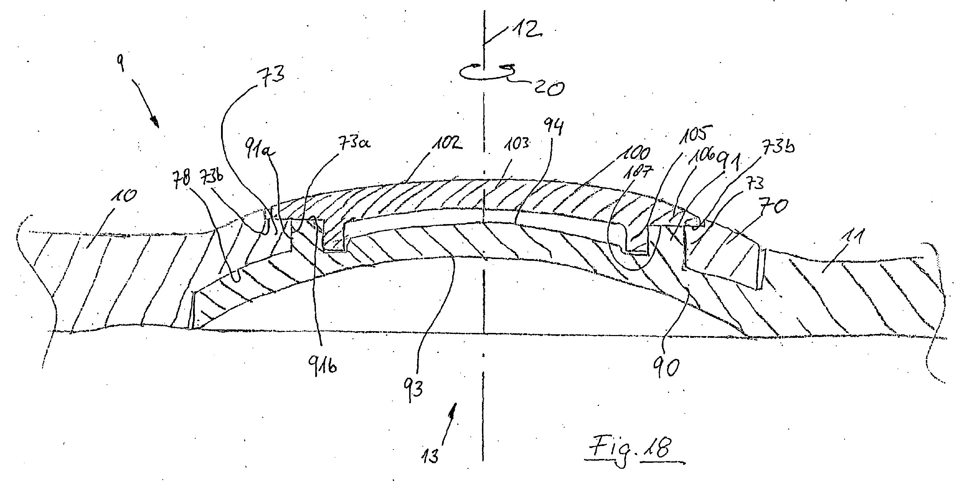

Eine weitere Ausführungsform der Gelenkeinrichtung 13 (Fig. 18) der Gelenkschiene 9 der erfindungsgemäßen Vorrichtung 1 ist ebenfalls als so genanntes Ringscheibengelenk mit einem Gelenkteller 90 und einem Gelenkring 70 aufgebaut. Der Gelenkteller 90 weist eine zur Fußhaut weisende Innenseite 93 und eine gegenüberliegende Außenseite 94 auf. Von der Außenseite 94 erstreckt sich ein Stück in Richtung weg vom Fuß des Patienten ein Ringsteg 91, welcher eine Ringstegaußenfläche 91a besitzt. Die Ringstegaußenfläche 91a dient zur radialen Führung des Gelenkrings 70 und wirkt spielfrei oder mit geringem Spiel mit einer Gelenkringinnenseite 73a zusammen.

Der Gelenkteller 90 ist wie in den vorangegangenen Ausführungsformen bevorzugt Kalotten-förmig, insbesondere Kugelkalotten-förmig ausgebildet und weist außerhalb des Ringstegs 91 einen Teilbereich der Außenseite 94 auf, welcher als Gelenkgleitfläche dient und mit einer korrespondierenden Gelenkgleitfläche 78 des Gelenkrings 70 zusammenwirkt. Die Gelenkgleitflächen haben eine Kalottenabschnitts-förmige Raumform. Bei der Ausführungsform gemäß der Fig. 18 ist die Höhe der Ringstufe 73 des Gelenkrings 70 derart gewählt, dass eine Oberseite 73b der Ringstufe mit einer Oberseite 91b des Ringstegs 91 bündig abschließt oder etwas übersteht. Somit ist durch das Zusammenwirken der Ringstege 91 und des Gelenkrings 70 mit deren Flächen 91a und 73a eine radiale Führung der Gelenkschienenschenkel 10, 11 gewährleistet. Die axiale Führung der Gelenkschienenschenkel 10, 11 zueinander wird über die Gleitfläche 78 und deren korrespondierenden Gleitflächenabschnitt der Außenseite 94 des Gelenktellers 90 in einer Richtung sichergestellt. In der zu dieser Richtung entgegengesetzten Richtung ist die axiale Festlegung der Gelenkschienenschenkel 10, 11 bzw. des Gelenkrings relativ zum Gelenkteller 90 wie folgt sichergestellt.

Die Verschlusseinrichtung 100 ist zusätzlich zu ihrer Verschlussfunktion der Gelenkeinrichtung 13 als Axiallager weitergebildet. Die Verschlusseinrichtung 100 weist einen Verschlusseinrichtungskörper 103 auf, welcher wie in den vorbeschriebenen Ausführungsformen im Wesentlichen die Gelenkeinrichtung 13 bedeckt und somit vor Verschmutzungen schützt. Vom Verschlusseinrichtungskörper 103 erstreckt sich gegenüberliegend von der Oberseite 102 ein Ringsteg 105, welcher im Durchmesser derart bemessen ist, dass er innerhalb des Ringstegs 91 des Gelenktellers 90 in den vom Ringsteg 91 umgrenzten Raum einsteckbar ist. Außerhalb des Ringstegs 105 weist die Verschlusseinrichtung 100 einen umlaufenden Vorsprung 106 auf, welcher in radialer Richtung derart bemessen ist, dass er den Ringsteg 91 radial ein Stück überragt und auf der Ringstufenoberseite 73b der Ringstufe spielfrei oder mit geringem axialen Spiel aufliegt. Somit dient eine Unterseite 107 des Ringvorsprungs 106, welche mit der Oberseite 73b der Ringstufe 73 zusammenwirkt, als axiales Widerlager. Damit durch eine solche Gelenkkonstruktion hohe Biegekräfte, welche die Gelenkschiene 9 ausüben muss, beschädigungsfrei und dauerhaft übertragen werden können, ist die Verschlussvorrichtung 100 zweckmäßigerweise mit dem Gelenkteller bzw. dessen Ringsteg 91 an einer oder mehreren deren Berührungsflächen verklebt oder anderweitig sicher befestigt.

Auch diese Gelenkeinrichtung 9 hat den besonderen Vorteil, dass die an den besonders empfindlichen Bereichen des Fußes anliegenden Flächen 93, z. B. im Bereich einer Pseudoexostose, glatt ausgebildet sind, d. h. dass keine Überstände oder Vertiefungen die dortigen Hautbereiche reizen. Zudem weist die Gelenkausführung gemäß Fig. 18 eine besonders hohe Biegesteifigkeit auf und ist auch durch missbräuchliches Überbiegen der Schiene nicht ohne weiteres zerlegbar, d. h. dass durch unsachgemäße Behandlung durch den Patienten, z. B. durch Tordieren, Überbiegen oder dergleichen es ausgeschlossen ist, dass die beiden Gelenkschienenschenkel 10, 11 voneinander trennbar sind.

Als bevorzugter Werkstoff für die Gelenkschienen hat sich insbesondere ein schlagzäher und hautverträglicher Kunststoff, z. B. aus der Gruppe der Polykarbonate, bewährt.

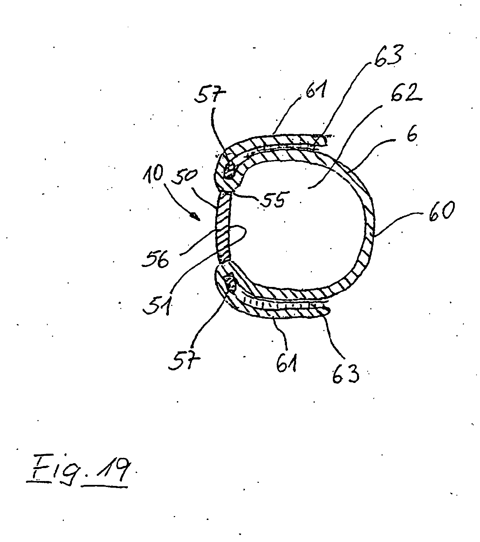

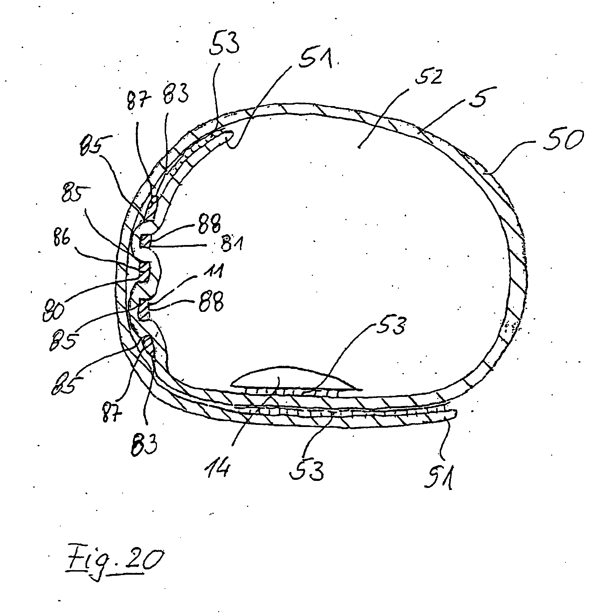

Im Folgenden wird anhand der Fig. 19 bis 21 die Befestigung der Gelenkschiene 9 an dem Patientenfuß und die Führung bzw. Fädelung der Ringbandagen 5, 6 durch die Schlitzöffnungen 55, 85 der Gelenkschienen 9 näher beschrieben.

Die Ringbandage 6 im Bereich des Großzehs (Fig. 19)dient als Befestigungseinrichtung und weist den Schlaufenkörper 60 sowie freie Enden 61 auf. Der Schlaufenkörper 60 umgrenzt zusammen mit dem ersten Gelenkschienenschenkel 10 einen Innenraum 62, in dem die Zehe des Patienten angeordnet ist. Die freien Enden 61 sind von der Innenseite 51 her durch die schlitzförmigen Öffnungen 55 geführt und um den Randsteg 57, diesen umschließend, herumgeschlagen. Somit kommt das freie Ende 61 der Ringbandage 6 benachbart zum Gelenkschienenschenkel 10 außenseitig mit dem Schlaufenkörper 60 zur Anlage. Das freie Ende 61 ist zweckmäßigerweise mittels eines Klettverschlusses 63 an der Außenseite des Schlaufenkörpers lösbar befestigt. Diese Art der Befestigung des freien Endes 61 kann auf beide freien Enden 61 der Ringbandage 6 angewendet werden. Es ist jedoch ggf. auch zweckmäßig, ein freies Ende 61 um einen Randsteg 57 herumzuschlagen und ggf. mit dem Schlaufenkörper 60 fest zu vernähen und nur ein weiteres freies Ende 61 mittels einer Klettverbindung oder dergleichen vergleichbare lösbare Flächenverbindung mit dem Schlaufenkörper 60 zu verbinden.

Die Ringbandage 5 dient als Befestigungseinrichtung und als Bandage zur Stützung und Wiederaufrichtung des Quergewölbes des Fußes und ist ebenfalls einen Schlaufenkörper 50 bildend mit dem zweiten Gelenkschienenschenkel 11 verbunden. Die Ringbandage 5 weist ebenfalls freie Enden 51 auf. Ein freies Ende 51 ist von der Innenseite 81 her durch die erste schlitzförmige Öffnung 85 nahe der Fußunterseite geführt und durchdringt diese, liegt außenseitig auf dem ersten Zwischensteg 88 auf und durchdringt die zweite Schlitzöffnung 85 von der Außenseite 80 her, liegt im Bereich des Mittelsteges 86 auf der Innenseite 81 auf und durchdringt die nächste schlitzförmige Öffnung 85 von der Innenseite 81 her hin zur Außenseite, liegt auf dem zweiten Zwischensteg 88 außenseitig an und durchdringt die letzte Schlitzöffnung 85 von der Außenseite 80 her nach innen. Das überstehende freie Ende 51 ist z.B. im Bereich des Ristes des Fußes anliegend angeordnet. Der restliche Bereich der Ringbandage 5, welcher nicht an der Fädelung durch den zweiten Gelenkschienenschenkel 11 beteiligt ist, ist den Fuß den Patienten umgebend um die Fußunterseite und die Fußoberseite geführt, überlappt den zweiten Gelenkschienenschenkel 11 im Bereich der Schlitzöffnungen 85 und ist im dem zweiten freien Ende 51 außenseitig am Schlaufenkörper 50 befestigt. Als Befestigungsmittel haben sich Klettverschlüsse, insbesondere Hakenklettverschlüsse oder Pilzklettverschlüsse 53 bewährt. Bei dieser Art der Fädelung der Ringbandage 5 durch den zweiten Gelenkschienenschenkel 11 und das anschließende Umgeben des gesamten Mittelfußes ist von besonderem Vorteil, dass die Randbegrenzungskanten 83 des Gelenkschienenschenkels 11 nicht direkt auf der Fußhaut aufliegen und somit eine Druckstellenbildung verhindert ist.

Gegebenenfalls kann es auch zweckmäßig sein, die zwei zentraler angeordneten Schlitzöffnungen 85 entfallen zu lassen und die Ringbandage 5 lediglich durch die äußersten Schlitzöffnungen 85 ohne den Umweg um den Mittelsteg 86 zu führen. Diese Ausführungsform hat den Vorteil, dass im Bereich des Gelenkschienenschenkels 11, in dem dieser am Fuß anliegt, weniger Unebenheiten und somit weniger Druckstellen am Fuß auftreten. Im Bereich des von Schlaufenkörper 50 umgebenen Innenraums 52, in dem der Mittelfußbereich des Patienten angeordnet ist, ist zweckmäßigerweise im Bereich der Fußunterseite des Patienten die Pelotte 14 mit der Ringbandage 5 ebenfalls über Klettverbindungselemente 53 verbunden.

Ein weiterer Vorteil dieser Art der Fädelung der Ringbandage 5. ist, dass zum Anlegen der Schiene 9 keine Fädelung durch die Öffnungen 85 des Gelenkschienenschenkels 11 notwendig ist, sondern lediglich die fertig gefädelte Ringbandage um den Fuß geschlagen werden muss und an dessen Unterseite festgezurrt und dort befestigt werden muss. Somit ist ein leichtes Einstellen oder Nachstellen der Spannung der Ringbandage 5 und somit deren Korrekturwirkung auf das Quergewölbe des Patientenfußes im Bereich des Mittelfußes ermöglicht.

Eine weitere Ausführungsform der Fädelung der Ringbandage 5 durch den zweiten Gelenkschienenschenkel 11 (gemäß Fig. 5) erfolgt derart, dass ein erstes freies Ende eine Schlitzöffnung 85 von der Innenseite 81 her durchgreifend, den Randsteg 87 im Bereich der Fußunterseite umgreifend geführt ist und außenseitig am Schlaufenkörper im Bereich der Fußunterseite mit diesem mittels Verbindungselementen 53 verbunden ist. Das andere freie Ende 51 ist im Bereich der Fußoberseite von der Innenseite 81 her durch eine randliche Schlitzöffnung 85 nach außen geführt, liegt außenseitig am ersten Zwischensteg 88 an und durchdringt eine weitere schlitzförmige Öffnung 85 in Richtung zur Innenseite 81 hin, liegt innenseitig am Mittelsteg 86 an, umschlingt diesen durch eine weitere Öffnung 85 und ist außenseitig in Richtung zur Fußoberseite hin, den Gelenkschienenschenkel 11 teilweise überlappend, geführt. Das freie Ende 51 kann somit mittels Verschlusselementen 53 außenseitig am Schlaufenkörper 50 befestigt werden.

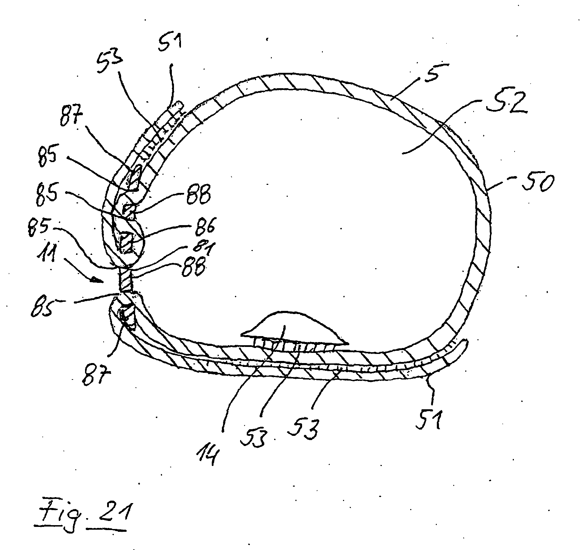

Die Ringbandagenführung gemäß Fig. 21 hat den Vorteil, dass die freien Enden in jedem Fall außenseitig am Schlaufenkörper 50 angeordnet sind, sodass zum Fuß hin keine Stufe in Höhe der Materialstärke zuzüglich der Verbindungselemente 51 entsteht und somit Druckstellen am Fuß vermieden werden. Auch bei dieser Ausführungsform ist es selbstverständlich möglich, das fußoberseitige freie Ende lediglich durch eine randliche Schlitzöffnung 85 zu führen, sodass dieses den Randsteg 87 umgreift und außenseitig auf dem Schlaufenkörper 50 befestigt werden kann. In diesem Fall entspricht die Fädelung bzw. Umschlingung der Ringbandage 5 der der Ringbandage 6 im Bereich des Großzehs. Auch kann bei dieser Ausführungsform im Bereich der Fußunterseite die Pelotte 14 mittels Klettelementen 53 an einer Innenseite des Schlaufenkörpers 50, insbesondere lösbar, befestigt werden.

Die nicht starre, in Grenzen schwimmende Anbindung der Vorrichtung 1 an die Innenseite des Fußes hat den Vorteil, dass sich die Vorrichtung 1 individuell an die anatomischen Randbedingungen anpassen kann, d.h. z.B. dass sich die Schwenkachse 12 der Gelenkschiene 9 nach dem Anlegen der Vorrichtung 1 selbsttätig fluchtend mit der anatomischen Gelenkachse des Großzehengrundgelenkes ausrichtet.

## Patentansprüche

1. Orthopädische Vorrichtung zur Korrektur von Zehen-Fehlstellungen,
mit einer Biegeschiene in der Form einer sehr dünnen Materialauflage zum Tragen in üblichem Schuhwerk,
mit einer Großzehen-Ringbandage und einer Mittelfuß-Ringbandage,
die als Befestigungseinrichtungen dienen und beide aus einem biegsamen, schmiegsamen zirkulär zugstarren Material bestehen,
wobei beim Tragen der Vorrichtung die Biegeschiene sich entlang der Fußinnenseite erstreckt, die Großzehen-Ringbandage die Großzehe und die Mittelfuß-Ringbandage den Mittelfuß zumindest teilweise umgibt,
mit der Wirkung, dass auf die Großzehe eine Korrekturkraft in Richtung zur Fußinnenseite hin ausgeübt wird und das Quergewölbe des Fußes durch die Mittelfuß-Ringbandage gestützt und wieder aufgerichtet wird, bei welcher Vorrichtung
a) die Biegeschiene als Gelenkbiegeschiene (9) mit einer Schwenkachse (12) ausgebildet ist und einen ersten Gelenkschienenschenkel (10) und einen zweiten Gelenkschienenschenkel (11) aufweist, deren gelenkseitige Enden durch ein Ringgelenk schwenkbar miteinander verbunden sind, und aus Kunststoff besteht, wobei das Ringgelenk durch einen Gelenkring (70) und einen Gelenkteller (90) gebildet ist und der Gelenkring (70) einstückig mit dem ersten Gelenkschienenschenkel (10) sowie der Gelenkteller (90) einstückig mit dem zweiten Gelenkschienenschenkel (11) ausgebildet ist;
b) als Befestigungseinrichtung für den ersten Gelenkschienenschenkel (10) die Großzehen-Ringbandage (6) und für den zweiten Gelenkschienenschenkel (11) die Mittelfuß-Ringbandage (5) vorgesehen sind;
wobei beim Tragen die orthopädische Vorrichtung die folgenden Wirkungen hat:
c1) die durch das Ringgelenk gebildete Schwenkachse (12) mit der anatomischen Gelenkachse des Großzehen-Grundgelenks in der Flexion-Extensions-Richtung fluchtet, so dass eine kinematisch weitgehend an die anatomischen Bedingungen des Großzehengrundgelenks angepasste Schwenkbewegung der Gelenkbiegeschiene ermöglicht wird;
c2) die Korrekturkraft F1 auf die Großzehe zu ihrer Lateralisierung in Richtung zur Fuβinnenseite hin durch die Biegeschiene (9) mit dem eine hohe Biegebelastbarkeit aufweisenden Ringgelenk ausgeübt wird, während die Großzehen-Ringbandage (6) zur Kraftübertragung dient.

2. Orthopädische Vorrichtung nach Anspruch 1, bei welcher der erste und der zweite Gelenkschienenschenkel (10, 11) aus einem durch Kohlenstoff-Fasern verstärkten Kunststoff bestehen.

3. Vorrichtung nach Anspruch 1 oder 2, bei welcher das Ringgelenk im Bereich des Großzehengrundgelenks an der Fußinnenseite (7) anliegend ausgebildet ist.

4. Vorrichtung nach einem der Ansprüche 1 bis 3, bei welcher die Gelenkschienenschenkel (10, 11) im Querschnitt linsenförmig ausgebildet sind.

5. Vorrichtung nach einem oder der Ansprüche 1 bis 4, bei welcher die gelenkseitigen Enden (16) der Gelenkschienen-schenkel (10, 11) eine Kugelkalottenraumform aufweisen und zueinander derart korrespondierend ausgebildet sind, dass die jeweils gelenkseitigen Enden (16) der Gelenkschienenschenkel (10, 11) formschlüssig passend ineinander legbar sind.

6. Vorrichtung nach einem der Ansprüche 1 bis 5, bei welcher die Gelenkschienenschenkel (10, 11) und das Ringgelenk in ihrer Raumform an die Fußkontur des Patienten angepasst sind.

7. Vorrichtung nach Anspruch 6, bei welcher die Gelenkschienen-schenkel (10, 11) in ihrer Längs- und Querrichtung eine gewölbte Raumform haben, wobei die Wölbung an die anatomischen Gegebenheiten eines Fußes angepasst sind.

8. Vorrichtung nach einem der Ansprüche 1 bis 7, bei welcher die Großzehen-Ringbandage (6) mit dem ersten Gelenkschienenschenkel (10) und die Mittelfuß-Ringbandage (5) mit den zweiten Gelenkschienenschenkel (11) verbunden ist.

9. Vorrichtung nach Anspruch 8, bei welcher zur Befestigung der Ringbandagen (5, 6) in den Gelenkschienenschenkeln (10, 11) parallel zu deren Längsrichtung verlaufende schlitzförmige Öffnungen (55, 85) vorgesehen sind, sodass ein Mittelsteg (56, 86), Randstege (57, 87) und Zwischenstege (88) gebildet sind.

10. Vorrichtung nach Anspruch 9, bei welcher die Ringbandagen (5, 6) Schlaufenkörper (60, 50) und freie Enden (51, 61) aufweisen.

11. Vorrichtung nach Anspruch 9 oder 10, bei welcher die Ringbandagen (5, 6) zum Mittelfuß bzw. zum Großzeh des Patienten hin in ihrer Führung bzw. Fädelung durch die Gelenkschienenschenkel (10, 11) stufenfrei angeordnet sind.

12. Vorrichtung nach einem der Ansprüche 9 bis 11, bei welcher die Mittelfuß-Ringbandage (5) vor dem Anlegen an den Patientenfuß vollständig in den zweiten Gelenkschienenschenkel (11) eingefädelt ist.

13. Vorrichtung nach einem der Ansprüche 1 bis 12, bei welcher zur axialen Festlegung des Gelenkrings (70) bezüglich des Gelenktellers (90) Verrastungselemente (92) vorgesehen sind, welche mit einer Stufe, insbesondere einer Ringstufe (73) zusammenwirken.

14. Vorrichtung nach einem der Ansprüche1 bis 13, bei welcher das Ringgelenk gegenüber einem Strumpf oder der Innenseite eines Schuhs mittels einer Verschlusseinrichtung (100) abgedeckt ist, welche mittels Rastmitteln (96, 101) mit der Gelenkbiegeschiene (9) verbunden ist.

15. Vorrichtung nach Anspruch 14, bei welcher die Verschlusseinrichtung (100) als Axiallager ausgebildet ist.

16. Vorrichtung nach einem der Ansprüche 1 bis 15, bei welcher die Gelenkschienenschenkel (10, 11) in ihrer Längserstreckung mit der Schwenkachse (12) einen Winkel α, β einschließen.

17. Vorrichtung nach Anspruch 16, bei welcher der Winkel α zwischen 75° und 115° beträgt.

18. Vorrichtung nach Anspruch 16 oder 17, bei welcher der Winkel β etwa 70° bis 110° beträgt.

## Claims

1. Orthopaedic device for correcting abnormal positions of toes,
with a flexible splint in the form of a very thin pad of material for wearing in conventional footwear,
with an annular binding for the big toe and an annular binding for the midfoot,
which bindings serve as fastening arrangements and are both made of a pliable, conformable material with circular tensional rigidity,
wherein the flexible splint extends along the inner aspect of the foot when the device is being worn, the annular binding for the big toe at least partially surrounds the big toe, and the annular binding for the midfoot at least partially surrounds the midfoot,
with the effect that a corrective force is exerted on the big toe in the direction of the inner aspect of the foot, and the transverse arch of the foot is supported by the annular binding for the midfoot and is realigned, in which device
a) the flexible splint is designed as a hinged flexible splint (9) with a pivot axis (12) and has a first hinged-splint branch (10) and a second hinged-splint branch (11), of which the hinge-side ends are connected pivotably to each other by an annular hinge, and is made of plastic, wherein the annular hinge is formed by a hinge ring (70) and a hinge plate (90), and the hinge ring (70) is formed integrally with the first hinged-splint branch (10) and the hinge plate (90) is formed integrally with the second hinged-splint branch (11);
b) the annular binding (6) for the big toe is provided as a fastening arrangement for the first hinged-splint branch (10), and the annular binding (5) for the midfoot is provided as a fastening arrangement for the second hinged-splint branch (11);
wherein the orthopaedic device has the following effects when being worn:
c1) the pivot axis (12) formed by the annular hinge is in alignment with the anatomical joint axis of the metatarso-phalangeal joint of the big toe in the flexion-extension direction, such that a pivoting movement of the hinged flexible splint is permitted that is substantially adapted kinematically to the anatomical conditions of the metatarso-phalangeal joint of the big toe;
c2) the corrective force F1 on the big toe, for its lateralization in the direction of the inner aspect of the foot, is exerted through the flexible splint (9) with the annular hinge having a high flexural load-bearing capacity, while the annular binding (6) for the big toe serves for force transmission.

2. Orthopaedic device according to Claim 1, in which the first and the second hinged-splint branches (10, 11) are made from a plastic reinforced with carbon fibres.

3. Device according to Claim 1 or 2, in which the annular hinge is designed bearing on the inner aspect (7) of the foot in the area of the metatarso-phalangeal joint of the big toe.

4. Device according to one of Claims 1 to 3, in which the hinged-splint branches (10, 11) are lens-shaped in cross section.

5. Device according to one of Claims 1 to 4, in which the hinge-side ends (16) of the hinged-splint branches (10, 11) have the three-dimensional shape of a universal ball joint and are designed corresponding to each other in such a way that the respective hinge-side ends (16) of the hinged-splint branches (10, 11) can be placed in each other with a form fit.

6. Device according to one of Claims 1 to 5, in which the hinged-splint branches (10, 11) and the annular hinge are adapted, in terms of their three-dimensional shape, to the foot contour of the patient.

7. Device according to Claim 6, in which the hinged-splint branches (10, 11) have a convex three-dimensional shape in their longitudinal and transverse directions, wherein the convexity is adapted to the anatomical features of a foot.

8. Device according to one of Claims 1 to 7, in which the annular binding (6) for the big toe is connected to the first hinged-splint branch (10) and the annular binding (5) for the midfoot is connected to the second hinged-splint branch (11).

9. Device according to Claim 8, in which, in order to fasten the annular bindings (5, 6), slit-shaped openings (55, 85) are provided in the hinged-splint branches (10, 11) and extend parallel to the longitudinal direction of the branches, such that a middle stay (56, 86), edge stays (57, 87) and intermediate stays (88) are formed.

10. Device according to Claim 9, in which the annular bindings (5, 6) have loop bodies (60, 50) and free ends (51, 61).

11. Device according to Claim 9 or 10, in which the annular bindings (5, 6) are routed or threaded steplessly, in the direction of the midfoot and big toe of the patient respectively, through the hinged-splint branches (10, 11).

12. Device according to one of Claims 9 to 11, in which the annular binding (5) for the midfoot is threaded completely into the second hinged-splint branch (11) before being applied to the patient's foot.

13. Device according to one of Claims 1 to 12, in which, for the purpose of securing the hinge ring (70) axially with respect to the hinge plate (90), latching elements (92) are provided which interact with a step, in particular an annular step (73).

14. Device according to one of Claims 1 to 13, in which the annular hinge is covered, with respect to a stocking or the inside of a shoe, by means of a closure arrangement (100), which is connected to the hinged flexible splint (9) by locking means (96, 101) .

15. Device according to Claim 14, in which the closure arrangement (100) is designed as an axial bearing.

16. Device according to one of Claims 1 to 15, in which the hinged-splint branches (10, 11), in their longitudinal extent, enclose an angle α, β with the pivot axis 12.

17. Device according to Claim 16, in which the angle α is between 75° and 115°.

18. Device according to Claim 16 or 17, in which the angle β is about 70° to 110°.

## Revendications

1. Dispositif orthopédique pour corriger des malpositions des orteils,
avec une attelle de flexion sous la forme d'une applique en matériau très mince destinée à être portée dans une chaussure usuelle,
avec un bandage annulaire pour le gros orteil et un bandage annulaire pour le milieu du pied, lesquels servent de dispositifs de fixation et se composent tous deux d'un matériau flexible, souple et résistant aux tractions circulaires,
l'attelle de flexion, lors du port du dispositif, s'étendant le long du côté intérieur du pied, le bandage annulaire pour le gros orteil entourant au moins en partie le gros orteil et le bandage annulaire pour le milieu du pied entourant au moins en partie le milieu du pied,
avec pour effet d'exercer une force de correction sur le gros orteil dans la direction du côté intérieur du pied, de supporter et de réaligner la voûte plantaire transversale au moyen du bandage annulaire pour le milieu du pied, dans lequel dispositif :
a) l'attelle de flexion est réalisée sous forme d'attelle de flexion articulée (9) avec un axe de pivotement (12) et présente une première branche d'attelle articulée (10) et une deuxième branche d'attelle articulée (11), dont les extrémités du côté de l'articulation sont connectées l'une à l'autre de manière pivotante par une articulation annulaire, et se compose de matière plastique, l'articulation annulaire étant formée par une bague d'articulation (70) et un disque d'articulation (90) et la bague d'articulation (70) étant réalisée d'une seule pièce avec la première branche d'attelle articulée (10) et le disque d'articulation (90) étant réalisé d'une seule pièce avec la deuxième branche d'attelle articulée (11) ;
b) le bandage annulaire pour le gros orteil (6) étant prévu en tant que dispositif de fixation pour la première branche d'attelle articulée (10) et le bandage annulaire pour le milieu du pied (5) étant prévu en tant que dispositif de fixation pour la deuxième branche d'attelle articulée (11) ;
lors du port, le dispositif orthopédique ayant les effets suivants :
c1) l'axe de pivotement (12) formé par l'articulation annulaire s'aligne dans la direction flexion-extension avec l'axe d'articulation anatomique de l'articulation principale du gros orteil de sorte qu'un mouvement de pivotement de l'attelle de flexion articulée adapté du point de vue cinématique essentiellement aux conditions anatomiques de l'articulation principale du gros orteil soit possible ;
c2) la force de correction F1 appliquée au gros orteil en vue de sa latéralisation dans la direction du côté intérieur du pied est exercée par l'attelle de flexion (9) avec l'articulation annulaire présentant une grande capacité de sollicitation en flexion, tandis que le bandage annulaire pour le gros orteil (6) sert au transfert de force.

2. Dispositif orthopédique selon la revendication 1, dans lequel la première et la deuxième branche d'attelle articulée (10, 11) se composent d'une matière plastique renforcée par des fibres de carbone.

3. Dispositif selon la revendication 1 ou 2, dans lequel l'articulation annulaire dans la région de l'articulation principale du gros orteil est réalisée de manière à s'appliquer contre le côté intérieur du pied (7).

4. Dispositif selon l'une quelconque des revendications 1 à 3, dans lequel les branches d'attelle articulée (10, 11) sont réalisées en forme de lentilles en section transversale.

5. Dispositif selon l'une quelconque des ou les revendications 1 à 4, dans lequel les extrémités du côté de l'articulation (16) des branches d'attelle articulée (10, 11) présentent une forme tridimensionnelle en forme de calotte sphérique et sont réalisées de manière à correspondre l'une avec l'autre de telle sorte que les extrémités (16) respectives du côté de l'articulation des branches d'attelle articulée (10, 11) peuvent être disposées de manière à s'ajuster par engagement positif l'une dans l'autre.

6. Dispositif selon l'une quelconque des revendications 1 à 5, dans lequel les branches d'attelle articulée (10, 11) et l'articulation annulaire sont adaptées de par leur forme tridimensionnelle au contour du pied du patient.

7. Dispositif selon la revendication 6, dans lequel les branches d'attelle articulée (10, 11) présentent, dans leur direction longitudinale et transversale, une forme tridimensionnelle courbe, la courbure étant adaptée aux données anatomiques d'un pied.

8. Dispositif selon l'une quelconque des revendications 1 à 7, dans lequel le bandage annulaire pour le gros orteil (6) est connecté à la première branche d'attelle articulée (10) et le bandage annulaire pour le milieu du pied (5) est connecté à la deuxième branche d'attelle articulée (11) .

9. Dispositif selon la revendication 8, dans lequel, pour la fixation des bandages annulaires (5, 6) dans les branches d'attelle articulée (10, 11), sont prévues des ouvertures (55, 85) en forme de fentes s'étendant parallèlement à leur direction longitudinale, de sorte qu'une nervure centrale (56, 86), des nervures marginales (57, 87) et des nervures intermédiaires (88) soient formées.

10. Dispositif selon la revendication 9, dans lequel les bandages annulaires (5, 6) présentent des corps en boucle (60, 50) et des extrémités libres (51, 61) .

11. Dispositif selon la revendication 9 ou 10, dans lequel les bandages annulaires (5, 6) en direction du milieu du pied ou du gros orteil du patient sont disposés sans gradin dans leur guidage ou leur enfilage à travers les branches d'attelle articulée (10, 11).

12. Dispositif selon l'une quelconque des revendications 9 à 11, dans lequel le bandage annulaire pour le milieu du pied (5) est complètement enfilé dans la deuxième branche d'attelle articulée (11) avant l'application sur le pied du patient.

13. Dispositif selon l'une quelconque des revendications 1 à 12, dans lequel, pour la fixation axiale de la bague d'articulation (70) par rapport au disque d'articulation (90), sont prévus des éléments d'encliquetage (92), lesquels coopèrent avec un gradin, en particulier un gradin annulaire (73).

14. Dispositif selon l'une quelconque des revendications 1 à 13, dans lequel l'articulation annulaire est recouverte par rapport à une chaussette ou au côté intérieur d'une chaussure au moyen d'un dispositif de fermeture (100), lequel est connecté par le biais de moyens d'encliquetage (96, 101) à l'attelle de flexion articulée (9).

15. Dispositif selon la revendication 14, dans lequel le dispositif de fermeture (100) est réalisé sous forme de palier axial.

16. Dispositif selon l'une quelconque des revendications 1 à 15, dans lequel les branches d'attelle articulée (10, 11) forment, dans leur étendue longitudinale, avec l'axe de pivotement (12), un angle α, β.

17. Dispositif selon la revendication 16, dans lequel l'angle α est compris entre 75° et 115°.

18. Dispositif selon la revendication 16 ou 17, dans lequel l'angle β est compris entre environ 70° et 110°.
